# EUROPEAN PATENT APPLICATION

(11) **EP 4 119 571 A1**
(43) Date of publication of application: **18.01.2023**
(21) Application number: 21767580.0
(22) Date of filing: 12.03.2021
(51) Int. Cl.: C07K 14/605, C12N 15/62, A61K 38/26, A61P 1/00, A61P 3/00

(54) **NOVEL BISPECIFIC PROTEIN AND USE THEREOF**

(30) Priority: 12.03.2020 KR 20200030727
(71) Applicant: SL Metagen, Seongnam-si, Gyeonggi-do 13488 (KR)
(72) Inventor: YANG, Sang In, Yongin-si Gyeonggi-do 16823 (KR); AN, In Bok, Seongnam-si Gyeonggi-do 13503 (KR)
(74) Representative: Dehns
(86) International application number: PCT/KR2021/003128
(87) International publication number: WO 2021/182928

(57) **Abstract**

The present invention relates to a novel bispecific fusion protein and a use thereof. More specifically, the present invention relates to: a bispecific fusion protein in which a GLP-1 analogue and a GLP-2 analogue are fused, or a bispecific fusion protein comprising a first fusion protein in which a GLP-1 analogue is linked to an antibody Fc region and a second fusion protein in which a GLP-2 analogue is linked to an antibody Fc region, wherein the bispecific fusion protein is produced by dimerization of the first fusion protein and the second fusion protein; and a pharmaceutical composition comprising same as an active ingredient, for the treatment of a disease or symptom requiring intestinal proliferation or metabolic diseases such as obesity, type 2 diabetes, and nonalcoholic steatohepatitis.

## Description

### TECHNICAL FIELD

The present invention relates to a novel bispecific protein and a use thereof, and more specifically, to a bispecific protein comprising a GLP-1 analogue and a GLP-2 analogue, and a use thereof.

### BACKGROUND ART

GLP-1 and GLP-2 are peptide hormones that are expressed as precursors called proglucagons and are produced through a series of tissue-specific protein cleavage processes. In particular, GLP-1 is produced and secreted by a particular nerve cell in the nucleus of the solitary tract of the brain stem and enteroendocrine L-cells in the small intestine upon intake of food, wherein the initial product, GLP-1 (1-37), is readily amidated and converted by cleavage into two equivalent biologically active forms (GLP-1 (7-36) amide and GLP-1 (7-37)). Since active GLP-1 acts to lower blood glucose levels in a glucose-dependent manner, it has been developed and used as a therapeutic agent for type 2 diabetes. Additionally, GLP-2 is a peptide with 33 amino acids generated by specific post-translational proteolytic cleavage of proglucagon in a process that also liberates GLP-1, and is produced by the intestinal endocrine L cell and various neurons in the central nervous system. Intestinal GLP-2 is co-secreted along with GLP-1 upon nutrient ingestion. When externally administered, GLP-2 is known to enhance intestinal growth and intestinal function, reduce bone breakdown and provide neuroprotection and currently is developed as therapeutic drugs for diseases such as short bowel syndrome, Crohn's disease, and osteoporosis.

As described above, GLP-1 previously garnered attention for its beneficial effect on glucose homeostasis in the treatment of type 2 diabetes patients (Gutniak et al., N. Engl. J. Med. 326: 1316-1322, 1992). However, similar to GLP-2, its role in the treatment of short bowel syndrome has been implied from the research finding that indicates decreased gastrointestinal secretion and motility (Kunkel et al., Neurogastroenterol. Motil. 23: 739-e328, 2011). In addition, it has been reported that the growth of the intestine and the fusion of crypt cells are promoted by the activation of the GLP1R signaling system by GLP-1 (Koehler et al., Cell Metabol. 21(3): 379-391, 2015). Based on this mechanism, Naia Pharmaceuticals Inc. has been conducting a phase 1 clinical trial for short bowel syndrome using GLP-1-XTEN, which has XTEN, a half-life extending peptide, linked to GLP-1. Furthermore, it has been reported that co-administration of GLP-1 and GLP-2 showed an additive effect on intestinal absorption compared to when each peptide was singularly administered (Madsen et al., Regul. Pept. 184: 30-39, 2013).

On the other hand, non-alcoholic fatty liver disease (NAFLD) is not a single disease, but rather includes various types of liver diseases ranging from simple fatty liver without inflammation, to chronic hepatitis and cirrhosis, and is also known to be closely linked to obesity and insulin resistance associated therewith. Non-alcoholic fatty liver disease may vary in disease severity ranging from mild fatty liver with no hepatocellular damage, to steatohepatitis with severe and persistent hepatocellular damage, and even to liver fibrosis or cirrhosis accompanied by ascites or jaundice, and is known to progress to liver cancer if further advanced, and is a Western disease that is rapidly increasing in incidence due to the prevalence of Western style diet. Most non-alcoholic fatty liver diseases are a mild disease but is not a symptom that can be safely ignored since if left untreated, one in four patients with severe fatty liver gradually progresses over time to cirrhosis (or liver fibrosis), which is a serious liver disease. The prevalence of non-alcoholic fatty liver disease varies according to characteristics of a population, and is reported to be 10-24% in normal people, and 58-74% in obese people. However, there is currently no drug approved as a therapeutic agent for non-alcoholic fatty liver disease or non-alcoholic steatohepatitis, and therefore, the development of a therapeutic agent for non-alcoholic fatty liver disease is considered a very urgent task.

Furthermore, there has been no therapeutic drug developed for metabolic syndrome such as nonalcoholic steatohepatitis that uses a dual agonist of GLP-1R and GLP-2R, which exhibits a superior effect compared to a single agonist GLP-1R.

### DISCLOSURE OF THE INVENTION

### TECHNICAL PROBLEM

To address various issues including the issues described above, the object of the present invention is to provide a novel bispecific protein that is capable of addressing drawbacks of the conventional GLP-2 analogue and GLP-1 analogue while retaining the functions of GLP-1 and GLP-2, and is effective in the treatment of metabolic syndrome such as non-alcoholic fatty liver disease. However, the protection scope of the present invention is not limited by the above objective.

### TECHNICAL SOLUTION

According to one aspect of the present invention, provided is a bispecific fusion protein having a GLP-1 analogue and a GLP-2 analogue fused to each other.

According to another aspect of the present invention, provided is a bispecific fusion protein comprising a first fusion protein having a GLP-1 analogue linked to an antibody Fc region, and a second fusion protein having a GLP-2 analogue linked to an antibody Fc region, wherein the bispecific fusion protein is generated by dimerization of the first fusion protein and the second fusion protein.

According to another aspect of the present invention, provided is a pharmaceutical composition comprising the bispecific fusion protein.

According to another aspect of the present invention, provided is a pharmaceutical composition for treating non-alcoholic steatohepatitis, comprising one or more such bispecific fusion protein as an active ingredient.

According to another aspect of the present invention, provided is a pharmaceutical composition for treating metabolic syndrome, comprising one or more such bispecific fusion protein as an active ingredient.

According to another aspect of the present invention, provided is a pharmaceutical composition for treating obesity, comprising one or more such bispecific fusion protein as an active ingredient.

According to another aspect of the present invention, provided is a pharmaceutical composition for treating type 2 diabetes, comprising one or more such bispecific fusion protein as an active ingredient.

According to another aspect of the present invention, provided is a pharmaceutical composition for treating liver fibrosis, comprising one or more such bispecific fusion protein as an active ingredient.

According to another aspect of the present invention, provided is a method of treating a disease or symptom requiring intestinal proliferation in a subject with a disease requiring intestinal proliferation, the method comprising administering a therapeutically effective amount of the bispecific fusion protein to the subject.

According to another aspect of the present invention, provided is a method of treating metabolic syndrome in a subject with metabolic syndrome, the method comprising administering a therapeutically effective amount of the bispecific fusion protein to the subject.

According to another aspect of the present invention, provided is a method of treating obesity in a subject with obesity, the method comprising administering a therapeutically effective amount of the bispecific fusion protein to the subject.

According to another aspect of the present invention, provided is a method of treating metabolic syndrome in a subject with type 2 diabetes, the method comprising administering a therapeutically effective amount of the bispecific fusion protein to the subject.

According to another aspect of the present invention, provided is a method of treating non-alcoholic fatty liver disease or non-alcoholic steatohepatitis in a subject with non-alcoholic fatty liver disease or non-alcoholic steatohepatitis, the method comprising administering a therapeutically effective amount of the bispecific fusion protein to the subject.

According to another aspect of the present invention, provided is a method of treating liver fibrosis in a subject with liver fibrosis, the method comprising administering a therapeutically effective amount of the bispecific fusion protein to the subject.

### ADVANTAGEOUS EFFECTS

The bispecific fusion protein of the present invention may significantly increase intestinal proliferation when administered in vivo, and thus may be utilized extremely efficiently in the treatment of diseases requiring intestinal proliferation, e.g., diseases such as short bowel syndrome, or metabolic diseases such as metabolic syndrome, obesity, type 2 diabetes, non-alcoholic fatty liver disease, and liver fibrosis.

### BRIEF DESCRIPTION OF THE DRAWINGS

FIG. 1 is a schematic diagram showing a rough structure of a bispecific fusion protein according to an embodiment of the present invention.
FIG. 2 is a schematic diagram showing interaction relationships between various GLP-1 and/or GLP-2 analogues, GLP-1 receptor, GLP-2 receptor, and other glucagon receptors.
FIG. 3a is a series of gel images showing the result of SDS-PAGE analysis after purification of various bispecific fusion proteins according to an embodiment of the present invention in non-reducing (NR) condition and reducing (R) condition.
M: size marker;
1: MG12-1(5 µg);
2: MG12-2(5 µg);
3: MG12-3(5 µg);
4: MG12-4(5 µg); and
5: MG12-5(5 µg).
FIG. 3b is a series of gel images showing the result of SDS-PAGE analysis after purification of GLP-2 homodimer (GLP-2-Fc homodimer, left) and MG12-5 (right) containing Knobs-into-Holes structure in non-reducing (NR) condition and reducing (R) condition.
M: size marker;
1 & 2: GLP-2-Fc homo(10 µg); and
3 & 4: MG12-5(10 µg).
FIG. 3c is a series of gel images (left) showing the result of SDS-PAGE analysis of MG12-6 according to an embodiment of the present invention in reducing and non-reducing conditions, and a chromatogram (right) showing a HPLC result of a purified protein.
M: size marker;
1: MG12-6; and
C: Ctrl (3 pg)-Herceptin.
FIG. 3d shows a series of gel images (left) showing the result of SDS-PAGE analysis of MG12-7 and MG12-8 according to an embodiment of the present invention in reducing and non-reducing conditions, and a chromatogram (right) showing a HPLC result of a purified protein.
M: size marker;
1: MG12-7;
2: MG12-8; and
3: Ctrl (3 pg)-Herceptin.
FIG. 3e shows gel images (top) showing the result of SDS-PAGE analysis of MG12-9 according to an embodiment of the present invention in reducing and non-reducing conditions, and a chromatogram (bottom) showing a HPLC result of a purified protein. The top of the chromatogram indicates size markers:
1: Reducing MG12-9;
2: Non-reducing MG12-9; and
3: Ctrl (3 pg)-BSA.
FIG. 4a is a graph showing biological activity as analyzed by luciferase reporter assay, of a GLP-1-Fc homodimer protein and a GLP-1 peptide according to an embodiment of the present invention.
FIG. 4b is a graph showing biological activity of MG12-1 according to an embodiment of the present invention as measured in comparison to a GLP-1 peptide.
FIG. 4c is a graph showing biological activity of MG12-3 according to an embodiment of the present invention as measured in comparison to a GLP-1 peptide.
FIG. 4d is a graph showing biological activity of MG12-4 according to an embodiment of the present invention as measured in comparison to a GLP-1 peptide.
FIG. 4e is a graph showing biological activity of MG12-5 according to an embodiment of the present invention as measured in comparison to a GLP-1 peptide.
FIG. 5a is a graph showing GLP-2 activity of a GLP-2-Fc homodimer protein according to an embodiment of the present invention, as analyzed by fluorescence spectroscopy, in comparison to a GLP-2 peptide.
FIG. 5b is a graph showing GLP-2 activity of an MG12-1 protein according to an embodiment of the present invention as analyzed by fluorescence spectroscopy in comparison to a GLP-2 peptide.
FIG. 5c is a graph showing GLP-2 activity of an MG12-3 protein according to an embodiment of the present invention as analyzed by fluorescence spectroscopy in comparison to a GLP-2 peptide.
FIG. 5d is a graph showing GLP-2 activity of an MG12-4 protein according to an embodiment of the present invention as analyzed by fluorescence spectroscopy in comparison to a GLP-2 peptide.
FIG. 5e is a graph showing GLP-2 activity of an MG12-5 protein according to an embodiment of the present invention as analyzed by fluorescence spectroscopy in comparison to a GLP-2 peptide.
FIG. 6a is a graph showing GLP-1 activity of a GLP-1-Fc homodimer according to an embodiment of the present invention as analyzed by a luciferase reporter assay.
FIG. 6b is a graph showing GLP-2 activity of a GLP-2-Fc homodimer protein according to an embodiment of the present invention, as analyzed by fluorescence spectroscopy.
FIG. 6c is a graph showing GLP-1 activity of an MG12-2 protein according to an embodiment of the present invention as analyzed by a luciferase reporter assay.
FIG. 6d is a graph showing GLP-2 activity of an MG12-2 protein according to an embodiment of the present invention, as analyzed by fluorescence spectroscopy.
FIG. 7a is a graph showing pharmacokinetics (PK) profiles of various bispecific fusion proteins according to an embodiment of the present invention when administered to an animal (rat) as analyzed by ELISA using GLP-1-Fc.
FIG. 7b is a graph showing pharmacokinetics (PK) profiles of various bispecific fusion proteins according to an embodiment of the present invention when administered to an animal (rat) as analyzed by ELISA using GLP-2-Fc.
FIG. 8a is a graph showing intestine weight after administration of various protein dimers according to an embodiment of the present invention (GLP-2-2G, GLP-2-Fc homodimer, and MG12-5) to model animals.
FIG. 8b is a graph showing body weight changes over 12 days after administration of various protein dimers according to an embodiment of the present invention (GLP-2-2G, GLP-2-Fc homodimer, and MG12-5) to model animals.
FIG. 8c is a graph showing energy absorption as measured prior to administration of various protein dimers according to an embodiment of the present invention (GLP-2-2G, GLP-2-Fc homodimer, and MG12-5) to model animals.
FIG. 8d is a graph showing energy absorption as measured at 1 week after administration of various protein dimers according to an embodiment of the present invention (GLP-2-2G, GLP-2-Fc homodimer, and MG12-5) to model animals.

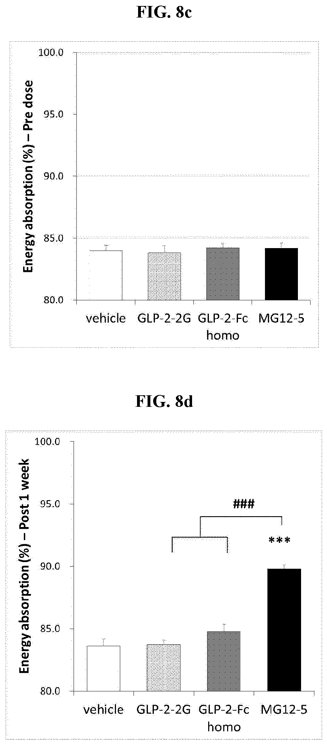

FIG. 8d is a graph showing energy absorption as measured at 2 weeks after administration of various protein dimers according to an embodiment of the present invention (GLP-2-2G, GLP-2-Fc homodimer, and MG12-5) to model animals.

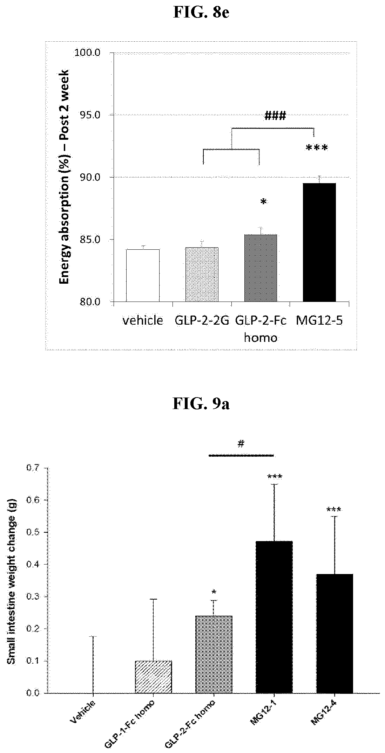

FIG. 9a is a graph showing small intestine weight changes 13 days after administration of various bispecific proteins (MG12-1 and MG12-4) according to an embodiment of the present invention and control groups (GLP-1-Fc homodimer and GLP-2-Fc homodimer) to model animals.

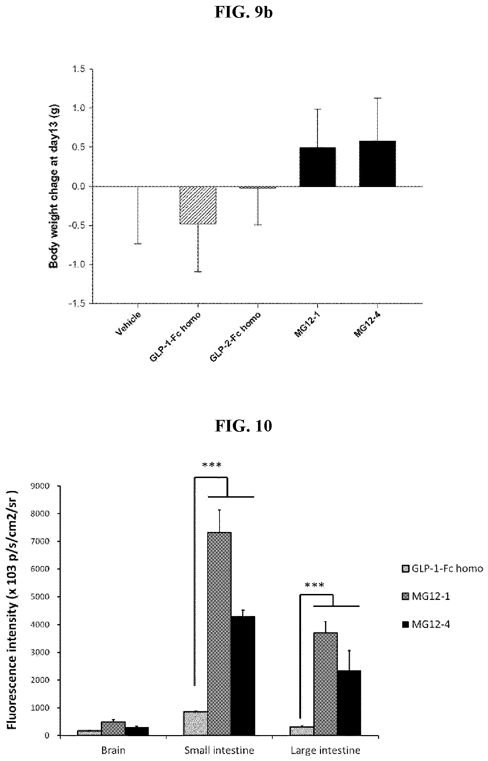

FIG. 9b is a graph showing body weight changes measured 13 days after administration of various bispecific fusion proteins (MG12-1 and MG12-4) according to an embodiment of the present invention and control groups (GLP-1-Fc homodimer and GLP-2-Fc homodimer) to model animals.
FIG. 10 is a graph showing the result of fluorescence intensity in the brain, the small intestine, and the large intestine of a model animal after administration of various bispecific fusion proteins (MG12-1 and MG12-4) according to an embodiment of the present invention and the control group (GLP-1-Fc homodimer) .

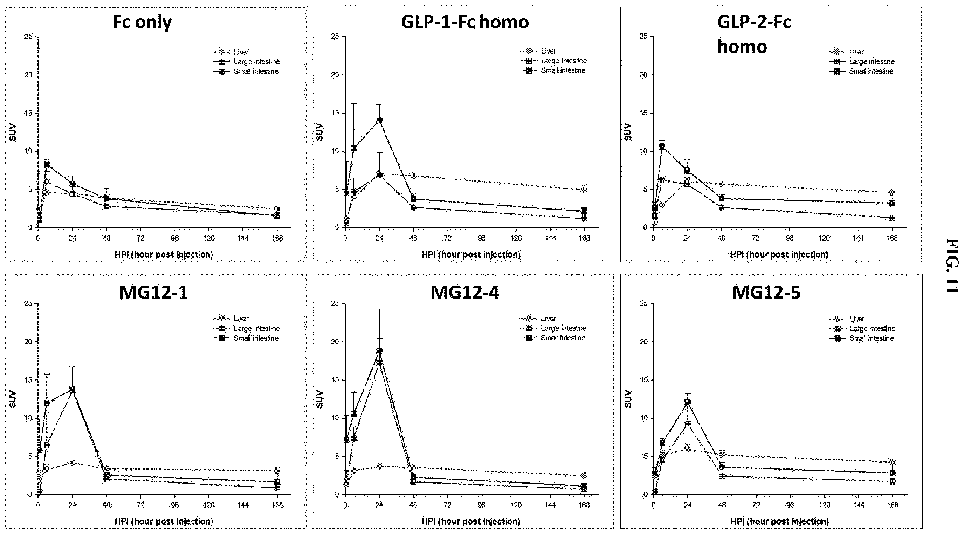

FIG. 11 is a series of graphs showing the distribution as analyzed by PET-MRI, of various bispecific fusion proteins (MG12-1, MG12-4 and MG12-5) according to an embodiment of the present invention and control groups (Fc only, GLP-1-Fc homodimer and GLP-2-Fc homodimer) in the liver, the small intestine, and the large intestine after administration to model animals.

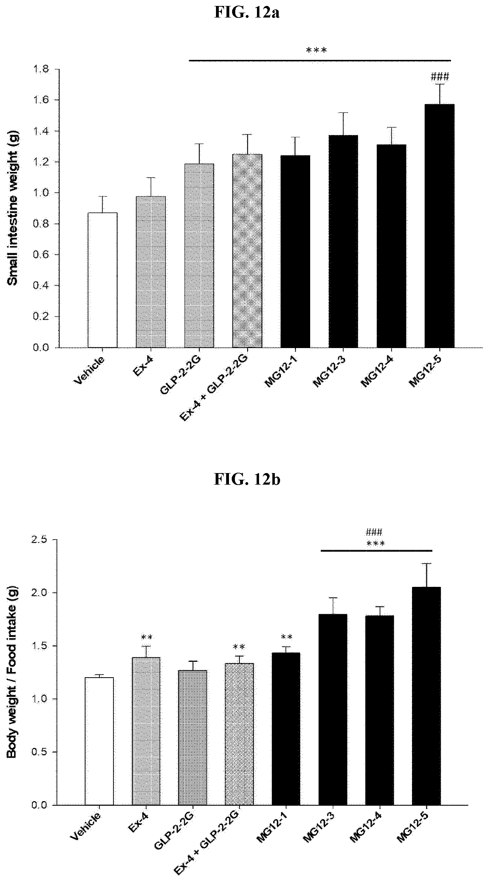

FIG. 12a is a graph showing the result of small intestine weight measurement after administration of various bispecific fusion proteins according to an embodiment of the present invention (MG12-1, MG12-3, MG12-4 and MG12-5) and Exendin 4(Ex-4), GLP-2-2G, and a combination of Exendin 4 and GLP-2-2G (Ex-4 + GLP-2-2G) to experiment animals for 12 days.
FIG. 12b is a graph showing the result of measurement of body weight increase vs. feed intake after administration of various bispecific fusion proteins according to an embodiment of the present invention (MG12-1, MG12-3, MG12-4 and MG12-5) and Exendin 4(Ex-4), GLP-2-2G, and a combination of Exendin 4 and GLP-2-2G (Ex-4 + GLP-2-2G) to experiment animals for 12 days.

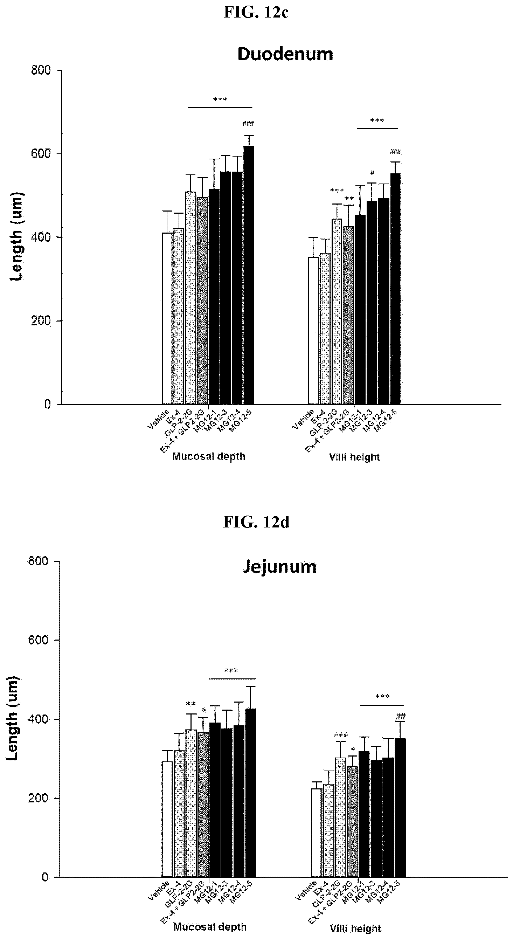

FIG. 12c is a graph showing the result of measurement of mucosal depth and villi height in the duodenum removed from experiment animals after administration of various bispecific fusion proteins according to an embodiment of the present invention (MG12-1, MG12-3, MG12-4 and MG12-5) and Exendin 4(Ex-4), GLP-2-2G, and a combination of Exendin 4 and GLP-2-2G (Ex-4 + GLP-2-2G) for 12 days.
FIG. 12d is a graph showing the result of measurement of mucosal depth and villi height in the jejunum removed from experiment animals after administration of various bispecific fusion proteins according to an embodiment of the present invention (MG12-1, MG12-3, MG12-4 and MG12-5) and Exendin 4(Ex-4), GLP-2-2G, and a combination of Exendin 4 and GLP-2-2G (Ex-4 + GLP-2-2G) for 12 days.

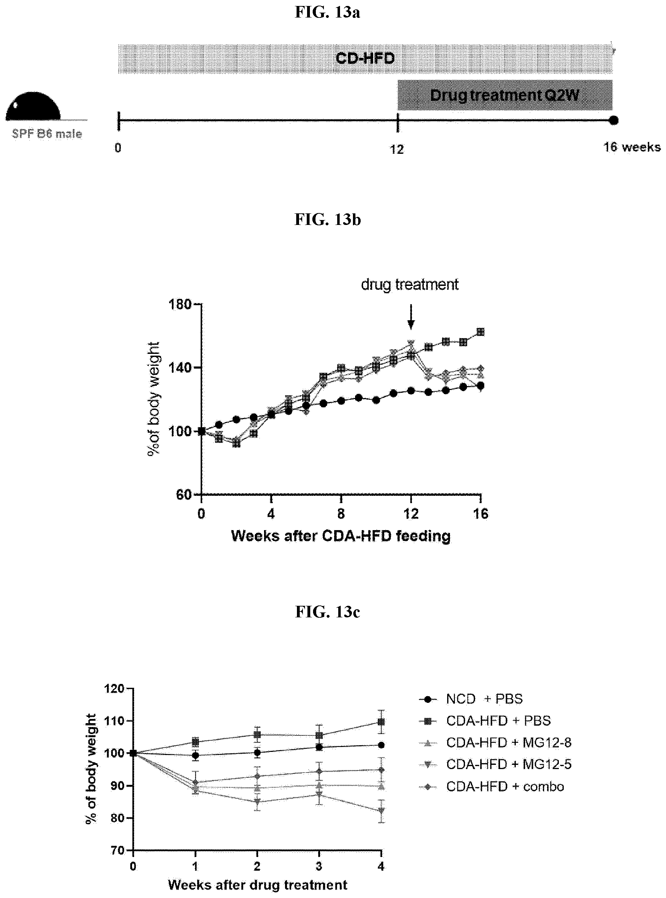

FIG. 13a is a schematic diagram schematically showing a MG12 dosing schedule according to an embodiment of the present invention for choline-deficient high-fat diet metabolic syndrome model animals.
FIG. 13b is a graph showing changes in body weight over time in an animal experiment performed according to an experiment schedule shown in FIG. 13a.
FIG. 13c is a graph showing relative changes in body weight over time after drug administration in an animal experiment performed according to an experiment schedule shown in FIG. 13a.

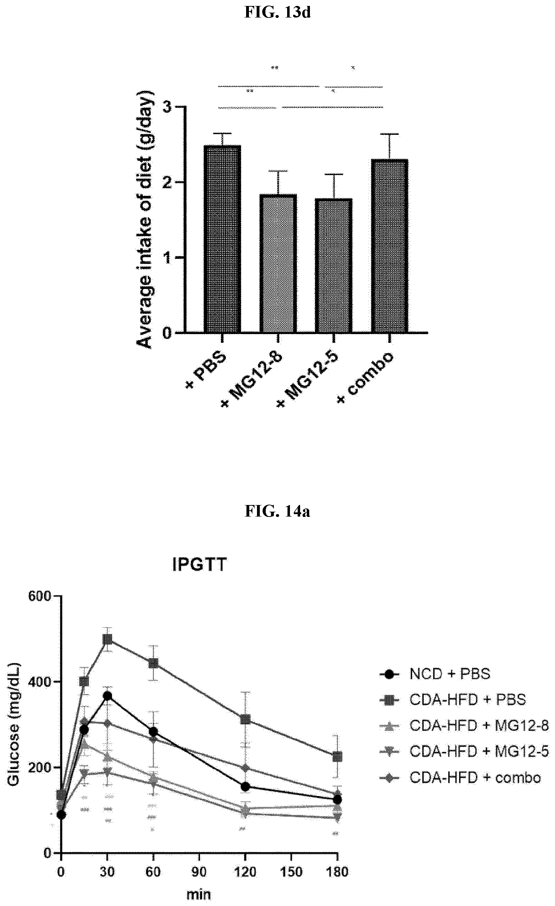

FIG. 13d is a graph showing the result of measurement of an amount of feed intake in an animal experiment performed according to an experiment schedule shown in FIG. 13a.
FIG. 14a is a graph showing the result of intraperitoneal glucose tolerance test performed for experimental groups, respectively used in the animal experiment performed according to the experimental schedule shown in FIG. 13a, and shows a record of blood glucose levels measured at a time interval for a total of 3 hours after intraperitoneal administration of glucose at a dose of 2 g/kg after 12 hours of feed restriction.

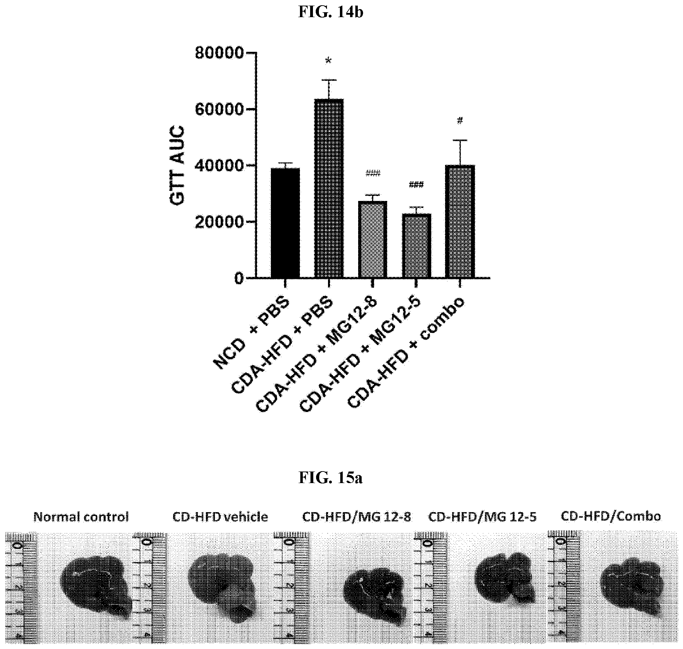

FIG. 14b is a graph showing the result of measuring the experiment result of FIG. 14a as an area under the curve (AUC) of blood glucose change.
FIG. 15a is a photograph of liver tissues excised from experimental animals of experimental groups, respectively sacrificed at the end of the animal experiment performed according to the experimental schedule shown in FIG. 13a.

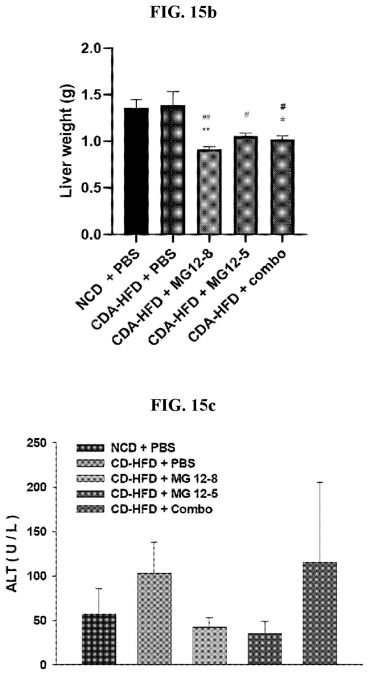

FIG. 15b is a graph showing the weight of liver tissues of experiment animals for experimental groups, respectively in FIG. 15a.
FIG. 15c is a graph showing the result of measurement of blood AST levels of experiment animals in the experimental groups, respectively in FIG. 15a.

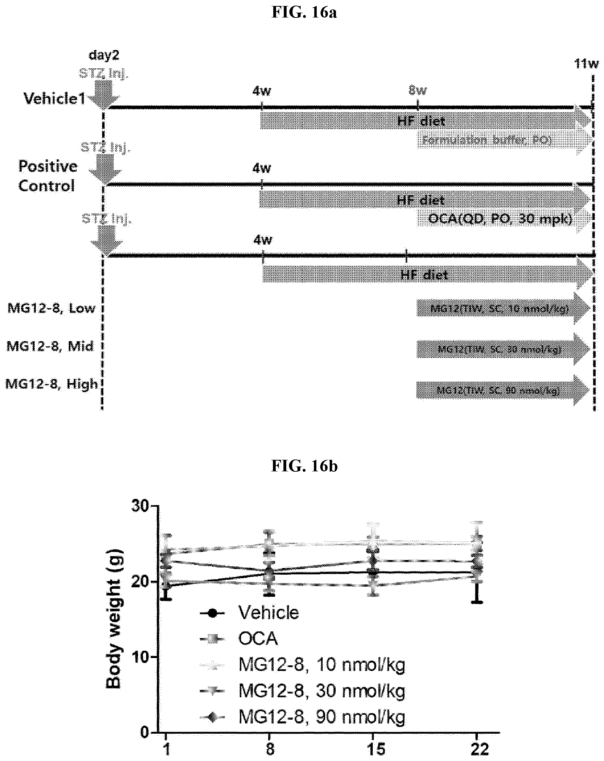

FIG. 16a shows diagrams showing a process of preparing severe NASH-induced model animals and a dosing schedule for an animal experiment to administer MG-12 according to an embodiment of the present invention, negative control group (vehicle) and obeticholic acid (OCA) as a positive control group.
FIG. 16b is a graph showing the result of measurement of changes in body weight over time for each experimental group after the eighth week in an animal experiment performed according to the experiment schedule illustrated in FIG. 16a.

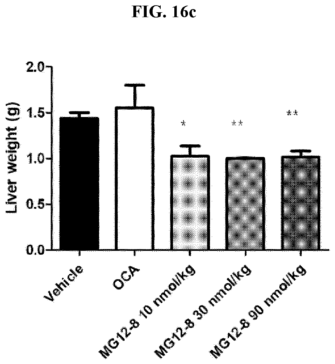

FIG. 16c is a graph showing the result of measurement of the weight of liver tissues excised from the experiment animals in the experimental groups, respectively sacrificed at the end of the animal experiment performed according to the experiment schedule illustrated in FIG. 16a.

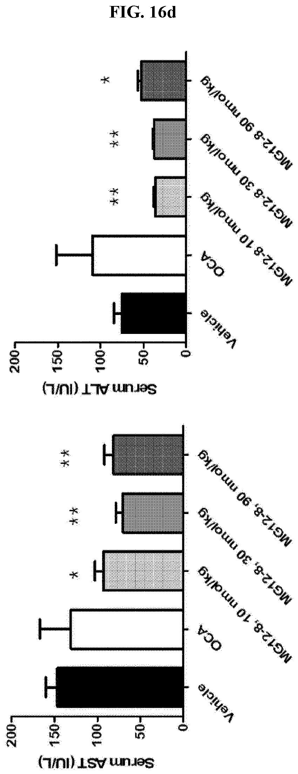

FIG. 16d is a series of graphs showing the result of measurement of blood AST (left) and ALT (right) levels for each experimental group in the animal experiment performed according to the experiment schedule illustrated in FIG. 16a.

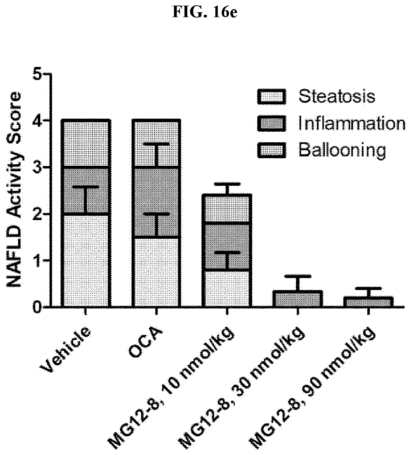

FIG. 16e is a graph showing the result of measurement of NAFLD activity score for each experimental group in the animal experiment performed according to the experiment schedule illustrated in FIG. 16a.

### BEST MODE FOR CARRYING OUT THE INVENTION

As used herein, the term "GLP-1" is an abbreviation of "glucagon-like peptide-1" and is a peptide hormone consisting of 30 or 31 amino acids derived by tissue-specific post-translational processing of proglucagon peptides. GLP-1 is produced and secreted by intestinal enteroendocrine L-cells and certain neurons within the nucleus of the solitary tract in the brain stem upon food intake. The initial product, GLP-1 (1-37), is readily amidated and converted by cleavage into two equivalent biologically active forms (GLP-1 (7-36) amide and GLP-1 (7-37)). Active GLP-1 contains two alpha-helical regions at amino acid positions 13-20 and 24-35 and a linker region connecting the two alpha-helical regions. GLP-1 has been, for its function to lower the blood glucose level in a glucose-dependent manner, developed and used as a therapeutic drug for type 2 diabetes. However, GLP-1 is rapidly degraded by dipeptidyl peptidase-4 (DPP-4) *in vivo,* and thus has an *in vivo* half-life of only 2 minutes and therefore as a natural peptide has an extremely limited effect.

As used herein, the term "GLP-2" is a 33 amino acids-long peptide that is generated along with GLP-1 by post-translational cleavage of proglucagon and is produced by the intestinal endocrine L cell and by various neurons in the central nervous system. Intestinal GLP-2 is co-secreted along with GLP-1 upon food intake. When administered, GLP-2 is known to enhance intestinal growth and intestinal function, reduce bone breakdown and provide neuroprotection and currently is developed as a therapeutic drug for diseases such as short bowel syndrome, Crohn's disease, and osteoporosis.

As used herein, the term "GLP-1 analogue" refers to a protein that biologically performs the function of GLP-1 and can mediate downstream signaling by binding to the GLP-1/Exendin-4 receptor, and is also referred to as "GLP-1 receptor agonists".

As used herein, the term "GLP-2 analogue" refers to a protein that biologically performs the function of GLP-2 and can mediate downstream signaling by binding to the GLP-2 receptor, and is also referred to as "GLP-2 receptor agonists".

As used herein, the term "fusion protein" refers to a recombinant protein in which two or more proteins or domains responsible for a specific function in the protein are linked so that each protein or domain is responsible for its inherent function.

As used herein, the term "half-life extending moiety" refers to a functional group linked to a recombinant protein to improve in vivo half-life of the recombinant protein. For such "half-life extending moiety", antibody Fc region (Capon et al., Nature 337: 525-531, 1989), PEG (Caliceti and Veronese, Adv. Drug Delivery Rev. 55: 1261-1277, 2003), XTEN (Schellenberger et al., Nat. Biotechnol. 27: 1186-1190, 2009), PAS (Pro-Ala-Ser, Schlapschy et al., Protein Eng. Des. Sel. 26: 489-501, 2013), ELP (elastine-like peptide, Floss et al., Trends Biotechnol. 28: 37-45, 2010), glycine-rich HAP (homo-amino-acid polymer, Schlapschy et al., Protein Eng. Des. Sel. 20: 273-284, 2007), GLK (gelatine-like protein, Huang et al., Eur. J. Pharm. Biopharm. 74(3): 435-441, 2010), and serum albumin (Sheffield et al., Cell Physiol. Biochem., 45(2): 772-782, 2018), and the like may be used. Additionally, such "half-life extending moiety" added to proteins are well-known through review journals and the like (Strohl, W. R., BioDrugs, 29(4): 215-239, 2015). Accordingly, the prior art documents and the review papers for the individual factors are incorporated herein by reference.

As used herein, the term "antibody Fc region" refers to a crystallized fragment among fragments generated when an antibody is cleaved with papain and interacts with a cell surface receptor called Fc receptor and a few proteins of the complement system. The Fc region represents a homodimer structure in which fragments containing the second and third constant regions (CH2 and CH3) of a heavy chain are linked by an intermolecular disulfide bond at the hinge region. The Fc region of IgG has a number of N-glycosylated sites, which are known to play an important role in the Fc receptor-mediated effects.

As used herein, the term "hybrid Fc region" refers to an Fc region peptide produced by a combination of parts of the Fc regions of various subtypes of immunoglobulins (Igs), and through combinations of these parts of the Fc regions, may be differentiated from wild-type Fc region in terms of binding affinity with Fc receptor and complement.

As used herein, the term "Exendin" is a peptide consisting of 39 amino acids isolated from the venom of the lizard Heloderma suspectum. Exendin 4 is 50% identical to GLP-1 in amino acid sequence, is a member of the glucagon peptide family, and is known to perform an equivalent role as GLP-1 as an agonist of the GLP-1 receptor. Exendin-4 is also referred to as "extenatide". Exendin 3 is a variant in which the second and third amino acids in Exendin 4 are substituted with serine and aspartic acid, respectively.

As used herein, the term "Lixisenatide" is one of GLP-1 receptor agonists, manufactured by Sanofi and marketed as a once-daily injection for the treatment of type 2 diabetes under the trade name Lyxumia in Europe and under the trade name Adlyxin in the United States.

The term "Albiglutide" used herein is one of the GLP-1 receptor agonists, which is marketed by GSK under the trade name of Eperzan in Europe and Tanzeum in the United States as a therapeutic drug for type 2 diabetes.

As used herein, the term "Liraglutide" is a subcutaneous injection-type GLP-1 receptor agonist marketed as a therapeutic drug for type 2 diabetes and obesity under the trade name "Victoza" by Novo Nordisk.

The term "Taspoglutide" used herein is a GLP-1 receptor agonist co-developed by Ipsen and Roche which is a therapeutic drug for type 2 diabetes, and is a GLP-1 derivative in which the 8^{th} and 35^{th} amino acids in GLP-1 (7-36) peptide are methylated and the terminal amino acid is amidated. However, when prepared in the form of a fusion protein with other peptides, the C-terminus can be a normal carboxyl group without amidation.

As used herein, the term "XTEN" is an unstructured peptide with low immunogenicity developed by Amunix, containing 6 amino acids added to improve the *in vivo* half-life of a protein drug, and typically has 144 a.a. as a unit and is composed of multiple units of amino acids thereof (US20100239554A1).

The term "Teduglutide" as used herein is a mutant in which the 2^{nd} amino acid, alanine (A) is substituted with glycine (G) in GLP-2, and is a GLP-2 analogue marketed under the trade name Gattex in the United States and Revestive in Europe as a therapeutic drug for short bowel syndrome.

The term "Glepaglutide" as used herein is a GLP-2 analogue with improved half-life, developed as a therapeutic drug for short bowel syndrome and is currently undergoing phase 3 clinical trials for short bowel syndrome.

As used herein, the term "GLP-2 analogue 10" is one of GLP-2 analogues in which the 11^{th} and 18^{th} amino acids are substituted with cysteine in GLP-2 and has a stabilized structure via an intramolecular crosslink with a lapidated crosslinker between thiol groups of the two substituted cysteines, and is also characterized in that 9 amino acids of the C-terminus of Exendin 4 (Yang et al., J. Med. Chem. 61: 3218-3223, 2018) are added at the C-terminus.

As used herein, the term "linker peptide" refers to an unstructured peptide used to prepare a fusion protein by linking two or more proteins or peptides having different biological activities.

As used herein, the term "malabsorption" refers to a disease caused by partial or complete non-absorption of nutrients in the intestinal tract, particularly in the small intestine. First, primary cause of malabsorption may include primary congenital abnormalities, such as deficiency of disaccharides-decomposing enzymes such as lactose and sugar, deficiency of digestive enzymes in the pancreas and small intestine, etc. and transmission disorder in the small intestine mucosa for glucose and vitamin B12, etc. and secondly, secondary cause of malabsorption may include persistent malabsorption caused by an intestinal disease, etc. as well as other causes such as deficiency in enzymes in the intestinal tract, changes in normal gut bacteria, insufficient digestion due to diseases in the pancreas, liver, gallbladder, etc. diseases in the intestinal tract due to parasites, stomachache, etc. and decreased absorption surface due to a bowel resection surgery, and the like. Risk factors include excessive alcohol consumption, bowel resection, family history of malabsorption or cystic fibrosis, and use of mineral oil or other laxatives, and the like.

The term "inflammatory bowel disease" used herein refers to a disease characterized by abnormal chronic inflammation of the intestinal tract undergoing repeated remission and relapse, and common examples thereof include ulcerative colitis and Crohn's disease, and its exact pathogenesis has not been elucidated yet. Diagnosis of inflammatory bowel disease is made by taking various findings into consideration, including clinical symptoms, endoscopic and histopathological findings, blood test findings, and radiological findings. Inflammatory bowel disease is a chronic disease characterized by repeatedly remitting and relapsing symptoms and the treatment goal thereof is to regulate symptoms, prevent complications and improve the quality of life, rather than full recovery of the disease.

As used herein, the term "ulcerative colitis" is one of inflammatory bowel diseases such as Crohn's disease and Behcet's disease, and refers to a disease characterized by chronic inflammation or ulceration of unknown cause in the large intestine. The exact cause of ulcerative colitis is still unknown. In most cases, symptoms undergo relapse and remission repeatedly, and as such, it is considered an incurable disease that is difficult to treat with modern medicine.

As used herein, the term "Bechete's disease" is an autoimmune disease characterized by breakdown of the mucous membranes in the body, especially the mucous membranes of the gastrointestinal tract, by autoimmunity. Bechet's disease is considered an incurable disease that manifests various symptoms where the location of lesion varies depending on the individual, and may be fatal if the lesion appears in areas such as the nervous system, gastrointestinal system, ocular system, and vascular system. Currently, there is no fundamental treatment for Bechet's disease.

As used herein, "short bowel syndrome (SBS)" is a malabsorption disorder that can be caused by surgical removal of a part of the small intestine or dysfunction of a segment of the intestine. For example, a majority of disorders are caused by Crohn's disease, inflammatory disorders of the digestive tract, volvulus, spontaneous twisting of the small intestine resulting in tissue death due to interruption of blood supply, tumors in the small intestine, wounds or traumas in the small intestine, necrotizing enterocolitis, bypass surgery for obesity treatment, and surgeries for a disease of the small intestine or removal of a damaged section of the small intestine. Also, some infants are born with short intestines.

As used herein, the term "metabolic syndrome" is a disease presumed to be caused by insulin resistance, and refers to a symptom characterized by two or more abnormal levels of cholesterol, blood pressure, and blood glucose. Metabolic syndrome is a cluster of risk factors for cardiovascular disease and type 2 diabetes, conceptualized as one disease group. It is a useful concept that can comprehensively account for insulin resistance (IR) and related complex and various metabolic abnormalities and clinical features. If left unattended, metabolic syndrome is known to increase the risk of developing cardiovascular diseases such as arteriosclerosis, myocardial infarction, and stroke, or type 2 diabetes.

### Detailed description of the invention:

According to one aspect of the present invention, provided is a bispecific fusion protein having a GLP-1 analogue and a GLP-2 analogue fused to each other.

The bispecific fusion protein may further comprise a half-life extending moiety, and the half-life extending moiety may be inserted between the GLP-1 analogue and the GLP-2 analogue or added to the N-terminus or C-terminus of the total fusion protein, and preferably, may be antibody Fc region, PEG, XTEN, PAS(Pro-Ala-Ser), ELP (elastin-like peptide), glycine-rich HAP (homo-amino-acid polymer), GLP (gelatin-like protein), or serum albumin.

The bispecific fusion protein may be not a proglucagon in which GLP-1 and GLP-2 naturally expressed *in vivo* are connected via an intervention peptide, or analogue thereof, but may be a fusion protein in which a GLP-1 analogue and a GLP-2 analogue are directly connected or the two peptides are connected via another form of linker peptide other than the intervention peptide.

According to one aspect, provided is bispecific fusion protein which comprises a first fusion protein having a GLP-1 analogue linked to an antibody fc region, and a second fusion protein having a GLP-2 analogue linked to an antibody fc region, and is formed by dimerization of the first fusion protein and the second fusion protein.

In the bispecific fusion protein, the GLP-1 analogue may be GLP-1, Exendin 3, Exendin 4, GLP-1/Exendin 4 hybrid peptide, GLP-1-XTEN, Exendin 4-XTEN, Lixisenatide, Albiglutide, Liraglutide, or Taspoglutide. Optionally, the GLP-1 analogue may be a GLP-1 tandem repeat having two GLP-1s connected via a linker peptide.

Additionally, in the bispecific fusion protein, at least one of the GLP-1 analogue and the GLP-2 analogue may be a tandem repeat, and the GLP-1 analogue and the GLP-2 analogue may have different numbers of repeats. Such a structure is introduced for asymmetry of the bispecific fusion protein, and a subsequent variation of the size of unit fusion protein constituting a heterodimer makes it easy to monitor the degree of homodimer formation, and thus enables quality control and simplifies the production process compared to PEGylation, which is well-known as the conventional half-life extension technique, and therefore, provides an advantage of reducing the production cost. The number of repeat units of the GLP-1 analogue and the GLP-2 analogue may be adjusted according to required binding affinities to GLP-1R and GLP-2R.

In the bispecific fusion protein, the GLP-1 may comprise the amino acid sequence represented by SEQ ID NO: 1 or 2.

In the bispecific fusion protein, the Exendin 3 may comprise the amino acid sequence represented by SEQ ID NO: 3.

In the bispecific fusion protein, the Exendin 4 may comprise the amino acid sequence represented by SEQ ID NO: 4.

In the bispecific fusion protein, the GLP-1/Exendin 4 hybrid may comprise the amino acid sequence represented by SEQ ID NO: 5.

In the bispecific fusion protein, the lixisenatide may comprise the amino acid sequence represented by SEQ ID NO: 6.

In the bispecific fusion protein, the Exendin 4-XTEN may comprise the amino acid sequence represented by SEQ ID NO: 7.

In the bispecific fusion protein, the albiglutide may comprise the amino acid sequence represented by SEQ ID NO: 8.

In the bispecific fusion protein, the liraglutide may comprise the amino acid sequence represented by SEQ ID NO: 9.

In the bispecific fusion protein, the taspoglutide may comprise the amino acid sequence represented by SEQ ID NO: 10.

In the bispecific fusion protein, the GLP-1 tandem repeat may comprise the amino acid sequence represented by SEQ ID NO: 11.

In the bispecific fusion protein, the antibody Fc region may be a hybrid antibody Fc region, and the hybrid antibody Fc region may consist of an amino acid sequence selected from the group consisting of SEQ ID NOs: 12 to 16. Additionally, the hybrid antibody Fc region may be one that is further modified so as to not cause adverse side effects, such as ADCC (antibody-dependent cell cytotoxicity) and CDC (complement-dependent cytotoxicity), when administered *in vivo.* Such a hybrid antibody Fc region may be one disclosed in Korean Patent No. 897938, or may be a hybrid Fc region variant consisting of amino acid sequence represented by SEQ ID NO: 14, in which the 18^{th} amino acid, threonine (T) is substituted with glutamine (Q) and the 196^{th} amino acid, methionine (M) is substituted with leucine (L) in a hybrid Fc region consisting of amino acid sequence represented by SEQ ID NO: 13, or may be a hybrid Fc region variant consisting of amino acid sequence represented by SEQ ID NO: 15, in which the 18^{th} amino acid, threonine (T) is substituted with glutamine (Q) and the 196^{th} amino acid, methionine (M) is substituted with leucine (L) in the hybrid Fc region consisting of the amino acid sequence represented by SEQ ID NO: 12.

In the bispecific fusion protein, the GLP-2 analogue may be GLP-2, glepaglutide, or GLP-2 analogue 10.

In the bispecific fusion protein, the GLP-2 may include an amino acid sequence of any one of SEQ ID NOs: 17 to 20. The peptide consisting of the amino acid sequence represented by SEQ ID NO: 18 is a wild-type human GLP-2 peptide, and a human GLP-2 variant consisting of the amino acid sequence represented by SEQ ID NO: 17 in which the 2^{nd} amino acid, alanine is substituted with glycine and is also known as teduglutide. Meanwhile, the GLP-2 variant consisting of the amino acid sequence represented by SEQ ID NO: 19 include GLP-2 variants (GLP-2 A2G, N16G, L17Q) in which the 2^{nd} amino acid, alanine (A) is substituted with glycine (G) and also, the 16^{th} amino acid, asparagine (N) is substituted with glycine (G) as well as the 17^{th} amino acid, leucine (L) is substituted with glutamine (Q). Such GLP-2 variants are known to suppress dimerization of GLP-2 or subsequent formation of aggregates during recombinant production, while maintaining the function as GLP-2 intactly. Optionally, a GLP-2 variant in which the 2^{nd} amino acid, alanine is substituted with glycine, and the 17^{th} amino acid, leucine is substituted with glutamine (A2G, L17Q, SEQ ID NO: 20) in the wild-type GLP-2 peptide has an analogous function as the GLP-2 analogue consisting of the above-described peptide consisting of the amino acid sequence represented by SEQ ID NO: 19, and thus may be used as a GLP-2 analogue in the present invention.

In the bispecific fusion protein, the Glepaglutide may comprise the amino acid sequence represented by SEQ ID NO: 21.

In the bispecific fusion protein, the GLP-2 analogue 10 may comprise the amino acid sequence represented by SEQ ID NO: 22.

In the bispecific fusion protein, the first fusion protein may comprise an amino acid sequence selected from the group consisting of SEQ ID NOs: 23 to 30.

In the bispecific fusion protein, the second fusion protein comprises an amino acid sequence selected from the group consisting of SEQ ID NOs: 31 to 36.

In the bispecific fusion protein, the first fusion protein may comprise a modified hybrid Fc region in which 10th amino acid, serine (S) is substituted with cysteine (C); and 22nd amino acid, threonine (T) is substituted with tryptophan (W) in the CH3 domain (Knob structure), and the second fusion protein comprises a modified hybrid Fc region in which the 5^{th} amino acid, tyrosine (Y) is substituted with cysteine (C); the 22^{nd} amino acid, threonine (T) is substituted with serine (S); the 24^{th} amino acid, leucine (L) is substituted with alanine (A); and the 63^{rd} amino acid, tyrosine (Y) is substituted with valine (V) in the CH3 domain (Hole structure), or the first fusion protein may comprise a modified hybrid Fc region in which the 5^{th} amino acid, tyrosine (Y) is substituted with cysteine (C); the 22^{nd} amino acid, threonine (T) is substituted with serine (S); the 24^{th} amino acid, leucine (L) is substituted with alanine (A); and the 63^{rd} amino acid, tyrosine (Y) is substituted with valine (V) in the CH3 domain (Hole structure), and the second fusion protein comprises a modified hybrid Fc region in which the 10^{th} amino acid, serine (S) is substituted with cysteine (C); and the 22^{nd} amino acid, threonine (T) is substituted with tryptophan (W) in the CH3 domain (Knob structure).

Optionally, the first fusion protein may comprise a modified hybrid Fc region in which the 22^{nd} amino acid, threonine (T) is substituted with tyrosine (Y) in the CH3 domain, and the second fusion protein may comprise a modified hybrid Fc region in which the 63^{rd} amino acid, tyrosine (Y) is substituted with threonine (T) in the CH3 domain, or the the second fusion protein may comprise a modified hybrid Fc region in which the 63^{rd} amino acid, tyrosine (Y) is substituted with threonine (T) in the CH3 domain, and the first fusion protein may comprise a modified hybrid Fc region in which the 63^{rd} amino acid, tyrosine (Y) is substituted with threonine (T) in the CH3 domain. However, the variation of the 63^{rd} amino acid may be expressed as Y86T etc. if following the IMGT (international ImMunoGeneTics information system) numbering rules (Lefranc et al., Dev. Comp. Immunol., 27: 55-77, 2003), rather than numbering based on the amino acid sequence of CH3 domain of human IgG1 having the amino acid sequence represented by SEQ ID NO: 69.

In the bispecific fusion protein, there may be one or more linker peptides inserted between fusion partners of the fusion proteins, that is, between peptides or domains. That is, in case of a bispecific fusion protein having the GLP-1 analogue and the GLP-2 analogue fused to each other, a linker peptide may be inserted between the GLP-1 analogue and the GLP-2 analogue. In case of a bispecific fusion protein created by dimerization of the first fusion protein and the second fusion protein, a linker peptide may be inserted between an antibody Fc region and the GLP-1 analogue in the first fusion protein and likewise, a linker peptide may be inserted between an antibody Fc region and the GLP-2 analogue in the second fusion protein. Here, the linker peptide may or may not include an N-glycosylated site, and more preferably, the first fusion protein may not include an N-glycosylated site in the linker peptide and the second fusion protein may include an N-glycosylated site in the linker peptide. Optionally, both of the first fusion protein and second fusion protein may not include an N-glycosylated site, or the first fusion protein may not include an N-glycosylated site in the linker peptide and the second fusion protein may include an N-glycosylated site in the linker peptide.

The linker peptide is EPKSSDKTHTCPPCP (SEQ ID NO: 37), EPKSCDKTHTCPPCP (SEQ ID NO: 38), GGGGSGGGGSGGGGSEPKSSDKTHTCPPCP (SEQ ID NO: 39), GGGGSGGGGSGGGGSEPKSCDKTHTCPPCP (SEQ ID NO: 40), AKATTAPATTRNTGRGGEEKKKEKEKEEQEERETKTPECP (SEQ ID NO: 41), GGGGSGGGGSGGGGSEKEKEEQEERTHTCPPCP (SEQ ID NO: 42), GGGGSGGGGSGGGGSAKNTTAPATTRNTTRGGEEKKKEKEKEEQEERTHTCPPCP (SEQ ID NO: 43), AAGSGGGGGSGGGGSGGGGS (SEQ ID NO: 44), GGGGSGGGGSGGGGS (SEQ ID NO: 45), GGSGG (SEQ ID NO: 46), GGSGGSGGS (SEQ ID NO: 47), GGGSGG (SEQ ID NO: 48), SEQ ID NO: (G₄S)ₙ (subunit: SEQ ID NO: 49, n is an integer of 1 to 10), (GGS)ₙ (n is an integer of 1 to 10), (GS)ₙ (n is an integer of 1 to 10), (GSSGGS)ₙ (subunit: SEQ ID NO: 50, n is an integer of 1 to 10), KESGSVSSEQLAQFRSLD (SEQ ID NO: 51), EGKSSGSGSESKST (SEQ ID NO: 52), GSAGSAAGSGEF (SEQ ID NO: 53), (EAAAK)ₙ (subunit: SEQ ID NO: 54, n is an integer of 1 to 10), CRRRRRREAEAC (SEQ ID NO: 55), A(EAAAK)₄ALEA(EAAAK)₄A (SEQ ID NO: 56), GGGGGGGG (SEQ ID NO: 57), GGGGGG (SEQ ID NO: 58), AEAAAKEAAAAKA (SEQ ID NO: 59), PAPAP (SEQ ID NO: 60), (Ala-Pro)n (n is an integer of 1 to 10), VSQTSKLTRAETVFPDV (SEQ ID NO: 61, PLGLWA (SEQ ID NO: 62), TRHRQPRGWE (SEQ ID NO: 63), AGNRVRRSVG(SEQ ID NO: 64), RRRRRRRR(SEQ ID NO: 65), GFLG(SEQ ID NO: 66), GSSGGSGSSGGSGGGDEADGSRGSQKAGVDE (SEQ ID NO: 67), or GSTSGSGKPGSGEGS (SEQ ID NO: 68).

According to another aspect of the present invention, provided is a pharmaceutical composition comprising the bispecific fusion protein.

The pharmaceutical composition may be utilized in the treatment of a disease or symptom requiring intestinal proliferation.

The disease or symptom requiring intestinal proliferation may be malabsorption, an inflammatory bowel disease, or short bowel syndrome.

In the pharmaceutical composition, the inflammatory bowel disease may be ulcerative colitis, Behcet's disease, or Crohn's disease.

The bispecific fusion protein according to an embodiment of the present invention has an effect of significantly improving the villus length and crypt depth in the small intestine, as well as increasing the length of the gastrointestinal tract, in particular the small intestine, when administered *in vivo,* and thus may be efficiently utilized in the treatment of a disease such as short bowel syndrome, that requires intestinal proliferation and functional improvement.

According to another aspect of the present invention, provided is a pharmaceutical composition for treating non-alcoholic steatohepatitis, comprising one or more such bispecific fusion protein as an active ingredient.

According to another aspect of the present invention, provided is a pharmaceutical composition for treating metabolic syndrome, comprising one or more such bispecific fusion protein as an active ingredient.

According to another aspect of the present invention, provided is a pharmaceutical composition for treating obesity, comprising one or more such bispecific fusion protein as an active ingredient.

According to another aspect of the present invention, provided is a pharmaceutical composition for treating type 2 diabetes, comprising one or more such bispecific fusion protein as an active ingredient.

According to another aspect of the present invention, provided is a pharmaceutical composition for treating liver fibrosis, comprising one or more such bispecific fusion protein as an active ingredient.

Exendin-4, which is a GLP-1 receptor agonist, was already proven to be effective in controlling blood glucose level and body weight in patients suffering from type 2 diabetes in clinical trials (DeFronzo et al., Diabetes Care 28: 1092-1100, 2005), the bispecific fusion protein according to an embodiment of the present invention may be used in the treatment of obesity-related diseases such as metabolic syndrome, as well as obesity and type 2 diabetes.

The composition may include a pharmaceutically acceptable carrier and may further include, other than the carrier, a pharmaceutically acceptable adjuvant, excipient or diluent.

The term "pharmaceutically acceptable" refers to a composition that is physiologically acceptable and does not normally cause gastrointestinal disorders, allergic reactions such as dizziness or similar reactions when administered to humans. Examples of the carrier, excipient, or diluent include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. Additionally, the composition may further include a filler, an antiaggregant, a lubricant, a wetting agent, a flavor, an emulsifier, a preservative, and the like.

Additionally, the pharmaceutical composition according to an embodiment of the present invention may be formed in a dosage form, using a method known in the art that enables rapid release, or sustained or delayed release of an active ingredient when administered to a mammal. The dosage form may include powders, granules, tablets, emulsions, syrups, aerosols, soft or hard gelatin capsules, sterile injectable solutions, and sterile packaged powders.

The composition according to an embodiment of the present invention may be administered via various routes, for example, oral, parenteral, for example, suppository, transdermal, intravenous, intraperitoneal, intramuscular, intralesional, nasal, intrathecal administration may be administered, and may also be administered using an implantable device for sustained release or continuous or repeated release. The dosing frequency may be, within a desired range, once or multiple times a day, and the duration of dosing also is not particularly limited.

The composition according to an embodiment may be made into an appropriate dosage form along with a commonly used, pharmaceutically acceptable carrier. Examples of the pharmaceutically acceptable carrier include carriers for parenteral administration such as water, a suitable oil, saline, aqueous glucose and glycol, and a stabilizer and preservatives may be further included. Examples of a suitable stabilizer include antioxidants such as sodium bisulfite, sodium sulfite or ascorbic acid. Examples of suitable preservatives include benzalkonium chloride, methyl- or propyl-paraben and chlorobutanol. Additionally, the composition according to the present invention may appropriately include, as necessary according to an administration method or dosage form thereof, a suspension, a solubilizer, a stabilizer, an isotonic agent, preservatives, an adsorption inhibitor, a surfactant, a diluent, an excipient, a pH adjuster, a soothing agent, a buffer, an antioxidant, and the like. Pharmaceutically acceptable carriers and agents suitable for the present invention, including those mentioned above, are described in detail in literature [Remington's Pharmaceutical Sciences, latest edition].

The dosage of the composition to a patient depends on many factors, including the patient's height, body surface area, age, the compound being administered, sex, time and route of administration, general health, and other drugs being administered simultaneously. A pharmaceutically active protein may be administered in an amount of 100 ng/body weight (kg) to 10 mg/body weight (kg), more preferably 1 to 500 µg/kg (body weight), and most preferably, at 5 to 50 µg/kg (body weight), and the dosage of the pharmaceutically active protein may be adjusted in consideration of the above factors.

According to another aspect of the present invention, provided is a method of treating a disease or symptom requiring intestinal proliferation in a subject with a disease requiring intestinal proliferation, the method comprising administering a therapeutically effective amount of the bispecific fusion protein to the subject.

In the treatment method, the disease or symptom requiring intestinal proliferation may be malabsorption, an inflammatory bowel disease, or short bowel syndrome, and may be preferably short bowel syndrome.

According to another aspect of the present invention, provided is a method of treating metabolic syndrome in a subject with metabolic syndrome, the method comprising administering a therapeutically effective amount of the bispecific fusion protein to the subject.

According to another aspect of the present invention, provided is a method of treating obesity in a subject with obesity, the method comprising administering a therapeutically effective amount of the bispecific fusion protein to the subject.

According to another aspect of the present invention, provided is a method of treating metabolic syndrome in a subject with type 2 diabetes, the method comprising administering a therapeutically effective amount of the bispecific fusion protein to the subject.

According to another aspect of the present invention, provided is a method of treating non-alcoholic fatty liver disease or non-alcoholic steatohepatitis in a subject with non-alcoholic fatty liver disease or non-alcoholic steatohepatitis, the method comprising administering a therapeutically effective amount of the bispecific fusion protein to the subject.

According to another aspect of the present invention, provided is a method of treating liver fibrosis in a subject with liver fibrosis, the method comprising administering a therapeutically effective amount of the bispecific fusion protein to the subject.

In the treatment method, the liver fibrosis may be liver fibrosis caused by chronic non-alcoholic steatohepatitis.

As used herein, the term "therapeutically effective amount" means an amount sufficient to treat a disease with a reasonable benefit/risk ratio applicable to a medical treatment, and an effective dose level depends on factors including the type and severity of a subject, age, sex, drug activity, sensitivity to drug, administration time, administration route and excretion rate, treatment duration, concomitantly used drugs, and other factors well known in the medical field. The therapeutically effective amount of the composition of the present invention may be 0.1 mg/kg to 1 g/kg, more preferably, 1 mg/kg to 500 mg/kg, and an effective dose may be adjusted depending on a patient's age, sex, and condition.

Between the two or more proteins or domains, a linker peptide that commonly has a flexible structure may be inserted. The linker peptide is EPKSSDKTHTCPPCP (SEQ ID NO: 37), EPKSCDKTHTCPPCP (SEQ ID NO: 38), GGGGSGGGGSGGGGSEPKSSDKTHTCPPCP (SEQ ID NO: 39), GGGGSGGGGSGGGGSEPKSCDKTHTCPPCP (SEQ ID NO: 40), AKATTAPATTRNTGRGGEEKKKEKEKEEQEERETKTPECP (SEQ ID NO: 41), GGGGSGGGGSGGGGSEKEKEEQEERTHTCPPCP (SEQ ID NO: 42), GGGGSGGGGSGGGGSAKNTTAPATTRNTTRGGEEKKKEKEKEEQEERTHTCPPCP (SEQ ID NO: 43), AAGSGGGGGSGGGGSGGGGS (SEQ ID NO: 44), GGGGSGGGGSGGGGS (SEQ ID NO: 45), GGSGG (SEQ ID NO: 46), GGSGGSGGS (SEQ ID NO: 47), GGGSGG (SEQ ID NO: 48), SEQ ID NO: (G₄S)ₙ (subunit: SEQ ID NO: 49, n is an integer of 1 to 10), (GGS)ₙ (n is an integer of 1 to 10), (GS)ₙ (n is an integer of 1 to 10), (GSSGGS)ₙ (subunit: SEQ ID NO: 50, n is an integer of 1 to 10), KESGSVSSEQLAQFRSLD (SEQ ID NO: 51), EGKSSGSGSESKST (SEQ ID NO: 52), GSAGSAAGSGEF (SEQ ID NO: 53), (EAAAK)ₙ (subunit: SEQ ID NO: 54, n is an integer of 1 to 10), CRRRRRREAEAC (SEQ ID NO: 55), A(EAAAK)₄ALEA(EAAAK)₄A (SEQ ID NO: 56), GGGGGGGG (SEQ ID NO: 57), GGGGGG (SEQ ID NO: 58), AEAAAKEAAAAKA (SEQ ID NO: 59), PAPAP (SEQ ID NO: 60), (Ala-Pro)n (n is an integer of 1 to 10), VSQTSKLTRAETVFPDV (SEQ ID NO: 61, PLGLWA (SEQ ID NO: 62), TRHRQPRGWE (SEQ ID NO: 63), AGNRVRRSVG(SEQ ID NO: 64), RRRRRRRR(SEQ ID NO: 65), GFLG(SEQ ID NO: 66), GSSGGSGSSGGSGGGDEADGSRGSQKAGVDE (SEQ ID NO: 67), or GSTSGSGKPGSGEGS (SEQ ID NO: 68).

According to another aspect of the present invention, the bispecific fusion protein may be produced through recombinant expression following transduction of a recombinant expression vector in a host cell, the recombinant expression vector including a first gene construct including a polynucleotide encoding the first fusion protein, and a second gene construct including a polynucleotide encoding the second fusion protein.

In such cases, the first gene construct and the second gene construct may be inserted in a single expression vector and expressed, or may be inserted in two separate expression vectors and expressed. For the former case, a vector may be designed such that each gene construct is operably linked to two different regulatory sequences, or such that two gene constructs are operably linked to a single regulatory sequence and the two gene constructs are connected by an internal ribosome entry site (IRES).

The phrase "operably linked to" as used herein refers to the association of a nucleic acid sequences of interest (e.g., in an in vitro transcription/translation system or in a host cell) is linked to the regulatory sequence in such a way that its expression can be achieved.

The term "regulatory sequence" as used herein includes promoters, enhancers, and other regulatory elements (e.g., polyadenylation signals). Regulatory sequences include sequences instructing a target nucleic acid to be consistently expressed in a variety of host cells, sequences instructing a target nucleic acid to be expressed only in specific tissue cells (e.g., tissue-specific regulatory sequences), and sequences instructing the expression to be induced by a specific signal (e.g., an inducible regulatory sequence). It can be understood by those skilled in the art that the design of an expression vector may vary depending on factors such as the selection of a host cell to be transformed, a desired protein expression level, and the like. The expression vector of the present invention may be introduced into a host cell and express the fusion protein. Regulatory sequences that enable expression in prokaryotic cells and eukaryotic cells are well known to those of ordinary skill in the art. As described below, such regulatory sequences include regulatory sequences responsible for transcription initiation and optionally, poly-A signals responsible for transcription termination and stabilization of transcripts. Additional regulatory sequences may include, in addition to transcription regulatory factors, translation enhancers and/or native-combined or heterologous promoter regions. For example, available regulator sequences enabling expression in mammalian host cells include CMV-HSV thymidine kinase promoter, SV40, RSV-promoter (Rous sarcoma virus), human kidney element 1α-promoter, glucocorticoid-inducible MMTV-promoter (Moloney mouse tumor virus), metallothionein-inducible or a tetracycline-inducible promoter, or amplifiers such as CMV amplifier or SV40-amplifier. For expression in nerve cells, possible use of neurofilament-promoter, PGDF-promoter, NSE-promoter, PrP-promoter, or thy-1-promoters is considered. These promoters are known in the art and disclosed in document (Charron, J. Biol. Chem. 270: 25739-25745, 1995). For expression in prokaryotic cells, a variety of promoters including lac-promoter, tac-promoter or trp promoter are disclosed. In addition to factors capable of transcription initiation, the above regulatory sequences may include transcription termination signals, such as SV40-poly-A site or TK-poly-A site, in downstream of a polynucleotide according to an embodiment of the present invention. In this document, suitable expression vectors are known in the relevant field, and examples thereof include Okayama-Berg cDNA expression vector, pcDV1(Parmacia), pRc/CMV, pcDNA1, pcDNA3 (Invitrogene), pSPORT1 (GIBCO BRL), pGX-27 (Patent No. 1442254), pX (Pagano et al., Science 255: 1144-1147, 1992), yeast 2-hybrid vector, e.g., pEG202 and dpJG4-5 (Gyuris et al., Cell 75: 791-803, 1995) or prokaryotic expression vector, e.g., lambda gt11 or pGEX (Amersham Pharmacia). Other than the nucleic acid molecules of the present invention, the vector may further include a polynucleotide encoding a secretion signal. The secretion signal is well known to those of ordinary skill in the art. Additionally, depending on the expression system used, a leader sequence capable of leading the fusion protein into cellular compartments is combined with a coding sequence of a polynucleotide according to an embodiment of the present invention, and preferably, the leader sequence is capable of secreting a decoded protein or a protein thereof directly to the surrounding of cytoplasm or cytoplasmic matrix.

In addition, the vector of the present invention can be prepared by, for example, standard recombinant DNA technology, and examples of the standard recombinant DNA technology include blunt-end and sticky-end ligation, restriction enzyme treatment to provide proper ends, dephosphorylation by alkaline phosphatase treatment in order to prevent improper bonding, and enzymatic ligation by T4 DNA ligase, and the like. By recombination of DNA encoding a signal peptide obtained by chemical synthesis or genetic recombination technology, and DNA encoding the bispecific fusion protein of the present invention with a vector including appropriate regulatory sequences, the vector of the present invention may be prepared. The vector containing the above regulatory sequences may be commercially purchased or prepared, and in one example of the present invention, pBispecific backbone vector (Genexine, Inc., Korea) or pAD15 vector were used as a backbone vector.

The expression vector may further include a polynucleotide encoding a secretion signal sequence, and the secretion signal sequence induce extracellular protein secretion, and may be tPA (tissue plasminogen activator) signal sequence, HSV gDs (Herpes simplex virus glycoprotein Ds) signal sequence or growth hormone signal sequence.

The expression vector according to an embodiment of the present invention may be an expression vector capable of expressing the protein in a host cell, and the expression vector may take any form, such as a plasmid vector, a viral vector, a cosmid vector, a phagemid vector, an artificial human chromosome, and the like.

### MODES OF CARRYING OUT THE INVENTION

Herebelow, the present invention may be described in greater detail through examples and experimental examples. However, the present invention is not limited to the examples and experimental examples disclosed below, but rather, can be implemented in various different forms. The following examples and experimental examples are provided to make the disclosure of the present invention complete and to provide a full understanding of the scope of the invention to those of ordinary skill in the art in the technical field to which the present invention belongs.

### Examples: Design of bispecific fusion proteins

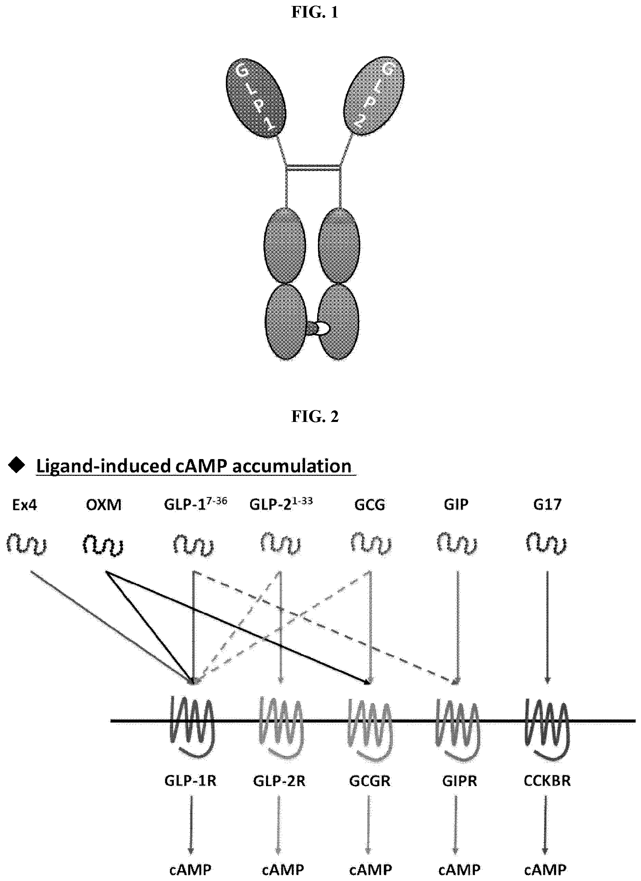

The present inventors designed various bispecific fusion proteins, including both a GLP-1 analogue and a GLP-2 analogue, as shown in FIG. 1 and Table 1.

In designing the bispecific fusion proteins, the main factor taken into consideration was that, as shown in FIG. 2, all of Exendin 4 (Ex4), oxyntomodulin (OXM), and GLP-2 as well as GLP-1 bind to a GLP-1 receptor, GLP-1R, whereas only GLP-2 binds to GLP-2R. Thus, in order to regulate the binding affinity of GLP-1 analogue to GLP-1R, the present inventors used a GLP-1 analogue including a glycan-binding domain so that the glycan can be attached to the linker region of the GLP-1 analogue. Also, a bispecific fusion protein (MG12-6) was designed such that a second fusion protein comprising GLP-2 includes a glycan linker while a first fusion protein including the GLP-1 analogue includes an unmodified linker that does not contain a glycan-binding site. Further, the present inventors designed bispecific fusion proteins MG12-7 and MG12-8, using a GLP-2 analogue in which the 17^{th} amino acid, alanine is substituted with glutamine in the GLP-2. In this case, the difference between MG12-7 and MG12-8 is that MG12-7 uses an A2G variant of SEQ ID NO: 1 as a GLP-1 analogue, whereas MG12-8 and MG12-9 use a tandem repeat of the A2G variants which is composed of two A2G variants connected via (G₄S)₆ linker.

**Table 1**

| Example | Name | GLP-1/2 analogue (SEQ ID NO) | Linker (SEQ ID NO) | Fc region | Overall configuration (SEQ ID NO) |
|---|---|---|---|---|---|
| 1 | MG12-1 | 1. GLP-1(1) | Glycan linker (43) | knob | first fusion protein (24) |
| | | 2. GLP-2(17) | Unmodified (42) | hole | second fusion protein (32) |
| 2 | MG12-2 | 1. GLP-2(17) | Glycan linker (43) | knob | second fusion protein (33) |
| | | 2. GLP-1(1) | Unmodified (42) | hole | first fusion protein (25) |
| 3 | MG12-3 | 1. Exendin 4(4) | Glycan linker (43) | knob hole | first fusion protein(26) |
| | | 2. GLP-2(17) | Unmodified (42) | | second fusion protein(32) |
| 4 | MG12-4 | 1. GLP-1/Exendin 4 hybrid(5) | Glycan linker (43) | knob | first fusion protein(27) |
| | | 2. GLP-2(17) | Unmodified (42) | hole | second fusion protein(34) |
| 5 | MG12-5 | 1. GLP-1/Exendin 4 hybrid(5) | Unmodified (42) | knob | first fusion protein(28) |
| | | 2. GLP-2(17) | Modified (42) | hole | second fusion protein(34) |
| 6 | MG12-6 | 1. GLP-2(17) | Glycan linker (43) | knob | second fusion protein(35) |
| | | 2. GLP-1/Exendin 4 hybrid(5) | Unmodified (42) | hole | first fusion protein(28) |
| 7 | MG12-7 | 1. GLP-1/Exendin 4 hybrid(5) | Glycan linker (43) | knob | first fusion protein(29) |
| | | 2. GLP-2(19) | Unmodified (39) | hole | second fusion protein (36) |
| 8 | MG12-8 | 1. GLP-1(11) | Unmodified (39) | knob | first fusion protein (30) |
| | | 2. GLP-2(19) | Unmodified (39) | hole | second fusion protein (36) |
| 9 | MG12-9 | 1. GLP-1(11) | Unmodified (39) | knob | first fusion protein (30) |
| | | 2. GLP-2(17) | Unmodified (42) | hole | second fusion protein(34) |

Also, for the bispecific fusion protein above, Knobs-into-Holes (KiH) technique was applied to permit preferential formation of heterodimers. In particular, the first fusion protein may be one (Knob) that has a hybrid Fc region in which, the 10^{th} amino acid, serine (S) is substituted with cysteine (C); and the 22^{nd} amino acid, threonine (T) is substituted with tryptophan (W) in the CH3 domain. The second fusion protein may be one (Hole) that has a Fc region in which the 5^{th} amino acid, tyrosine (Y) is substituted with cysteine (C); the 22^{nd} amino acid, threonine (T) is substituted with serine (S); the 24^{th} amino acid, leucine (L) is substituted with alanine (A); and the 63^{rd} amino acid, tyrosine (Y) is substituted with valine (V) in the CH3 domain. Here, the amino acid positions at which such variations have occurred are with respect to a reference sequence (amino acid sequence of human IgG1 CH3 domain of SEQ ID NO: 69). Even if additional variations such as addition, deletion or substitution of amino acids occur at a site unrelated to the Knobs-into-Holes structure on the CH3 domain, one having variations of amino acids corresponding to the positions based on the reference sequence may be used. Optionally, the Knobs-into-Holes structure may be introduced by other amino acid changes known in the art. Such variations are described in detail in prior art document (Wei et al., Oncotarget 2017, 8(31): 51037-51049; Ridgway et al., Protein Eng. 1996, 9(7): 617-621; Carter, P., J. Immunol. Methods 2001, 48(1-2): 7-15; Merchant et al., Nat. Biotechnol. 1998, 16(7): 677-681). Such selective variations include, for example, a Knob structure in which threonine, the 22^{nd} amino acid of the CH3 domain of the first fusion protein, is substituted with tyrosine, and a Hole structure in which tyrosine, the 63^{rd} amino acid in the CH3 domain of the second fusion protein is substituted with threonine, and through a combination of these structures, a bispecific dimeric fusion protein may be generated. Alternatively, the Knobs-into-Holes structure may be formed by introducing a Hole structure into the first fusion protein and introducing a Knob structure into the second fusion protein. After synthesizing gene constructs encoding the first fusion protein and the second fusion protein of the bispecific fusion protein of Examples 1 to 5 by amplification using PCR and site-directed mutagenesis primers, the gene constructs were then inserted into pAD15 vector (Genexine, Inc., Korea) to prepare expression vectors.

Transient expression of the vector constructs prepared as described above was performed using ExpiCHO kit by Thermo Fisher. Specifically, in ExpiCHO-S cells, after mixing the vector constructs prepared as described above and the ExpiFectamine reagent included in the kit, the resulting mixture was incubated for 1 day in an incubator with 8% CO₂ at 37°C, and after lowering the temperature to 32°C, incubated until the 7^{th} day.

The supernatant obtained from the incubation were appropriately diluted with the fusion proteins of Examples 1 to 5 (respectively, 'MG12-1', 'MG12-2', 'MG12-3', 'MG12-4', and 'MG12-5') purified through protein A column and secondary column with 4X LDS sample buffer and water for injection to a final volume of 3-10 µg/20 µL. For reducing condition samples, each substance to be analyzed, 4X LDS sample buffer, 10X reducing agent, and water for injection were appropriately diluted to a final volume of 3-10 µg/20 µL and heated for 10 minutes on 70 °C heating block. 20 µL of the prepared sample was loaded into each well of gel fixed on a pre-installed electrophoresis equipment. The size markers were loaded at 3-5 µL/well. After setting the power supply to 120 V and 90 minutes, electrophoresis was performed. After the electrophoresis was completed, the gel was separated and stained using a staining solution and a de-staining solution, and the results thereof were analyzed.

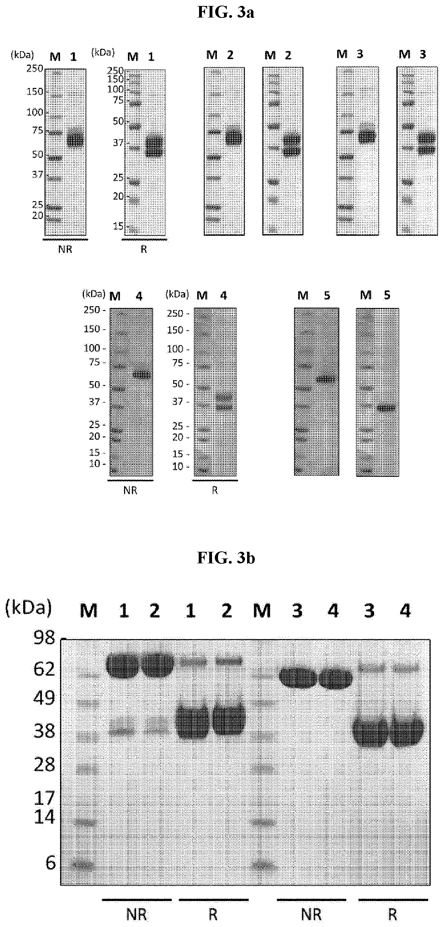

As a result of the analysis, as shown in FIG. 3a, all bispecific fusion proteins were observed at a site between 50 and 75 kDa under non-reducing conditions and 37 kDa under reducing conditions. Among the bispecific fusion protein of the present invention in a heterodimeric form, in the case of the fusion proteins of Examples 1 to 4 containing a sugar chain at one hinge, monomers having two different sizes were observed under reducing conditions, and in the case of the fusion protein (MG12-5) of Example 5 not containing a sugar chain at hinge, a monomer having one size was observed.

In addition, the present inventors conducted SDS-PAGE analysis on the GLP-2 homodimer fused with Fc not containing the Knobs-into-Holes (KiH) structure (GLP-2-Fc homodimer, SEQ ID NO: 25) and MG12-5 containing the Knobs-to-Holes structure under the same reducing/non-reducing conditions as above, respectively.

As a result, as shown in FIG. 3b, it was found that in the case of MG12-5 having a KiH structure, compared to GLP-2-Fc homodimer not containing such a structure, formation of monomeric impurities was significantly inhibited under non-reducing conditions.

In addition, the present inventors synthesized polynucleotides encoding MG12-6 to MG12-9, and then transfected HEK293F cells using the N293F vector system (YBiologics) for temporary expression in animal cells to perform plasmid DNA transfection. For nucleic acid preparation, 25 µg of plasmid DNA was mixed with 3 ml of media, and then mixed with 25 µg of 2 mg/ml PEI (Polyethylenimine, PolyPlus, USA). The reaction solution was left at room temperature for 15 minutes, then put into 80-1000 ml of culture solution cultured at 1×10⁶ cells/ml and incubated for 24 hours at 120 rpm, 37°C and 8% CO₂. 24 hours after DNA transfection, a nutrient supplement medium component (Soytone, BD, USA) was added to a final concentration of 10 g/L. After culturing for 7 days, the cell culture medium was centrifuged at 5,000 rpm for 10 minutes, and the supernatant was collected. Then, Protein A resin was filled in a column and prepared by washing with 1× DPBS, and the collected supernatant was bound with resin at 4°C at a rate of 0.5 ml/min, and proteins were eluted with 0.1 M glycine. To replace the buffer, the eluted solution was put into a dialysis tube (GeBAflex tube, Geba, Israel) and dialyzed with 1× DPBS at 4°C, and the material obtained therefrom was formulated in PBS (pH 7.4) buffer.

The MG12-6 to MG12-9 thus obtained were also subjected to SDS gel electrophoresis under reducing and non-reducing conditions as described above. As a result, as shown in FIGs. 3c to 3e, it was confirmed that the heterodimeric fusion proteins according to an embodiment of the present invention were normally expressed.

In conclusion, it was confirmed that all bispecific fusion proteins according to the present invention could be normally produced and purified through SDS-PAGE, and in particular, as demonstrated through the comparison of MG12-5 and GLP-2-Fc homodimer, in case of the bispecific dimeric protein to which the KiH structure was applied, compared to when such a structure was not applied, the formation of impurities in monomeric form was significantly reduced.

### Experimental Example 1: Confirmation of GLP-1 in vitro activity

The present inventors investigated *in vitro* activity of GLP-1 of the bispecific fusion proteins prepared in the above-described Examples by cAMP analysis. Specifically, in order to evaluate the degree of cAMP induction by GLP-1 specific reaction, a transformed cell line (GLP1R_cAMP/luc) was prepared so as to co-express a GLP-1 receptor in a cAMP-specific luciferin-expressing cell line. After the cells were thawed and properly maintained, the cells were detached from the flask by addition of 0.05% TE (Trypsin EDTA) and the number of viable cells was counted. The number of cells required for activity evaluation was collected and washed, diluted with 0.5% FBS, DMEM/high glucose medium, and seeded at 2×10⁴ cells/80 µL/well. After culturing the cells in a 37°C, 5% CO₂ incubator for about 16 hours, the cells were treated with 20 µL/well of a test solution of various concentrations to be evaluated and allowed to react in a 37°C, 5% CO₂ incubator for 5 hours. After the reaction was complete, the plate was treated with Bright-Glo^{™} assay reagent at 100 µL/well, and then was allowed react at room temperature for 2 minutes. After the reaction was completed, the plate was inserted into a Luminometer and the degree of bioluminescence was measured.

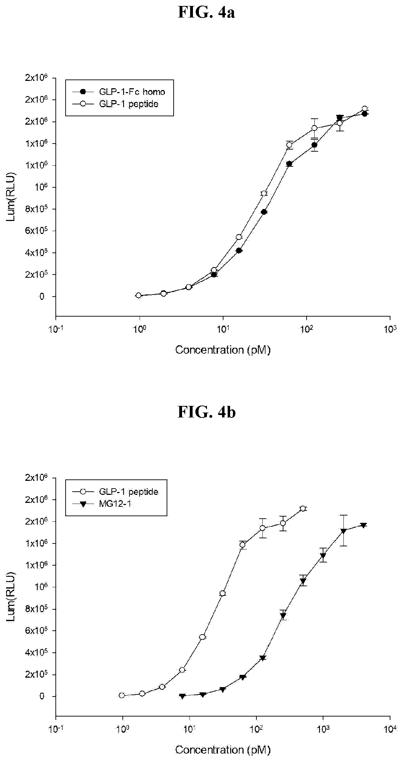

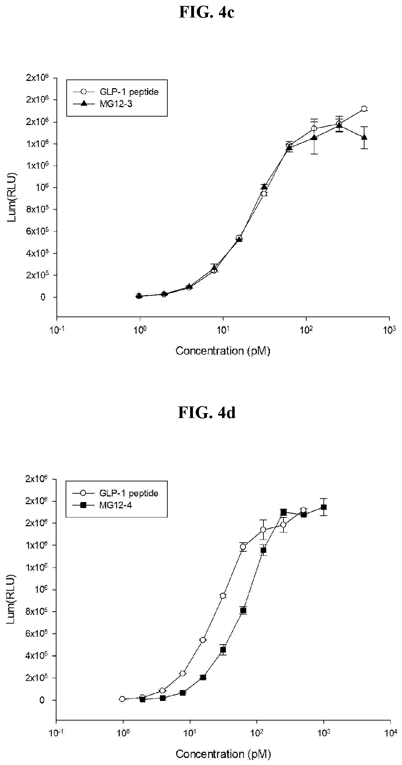

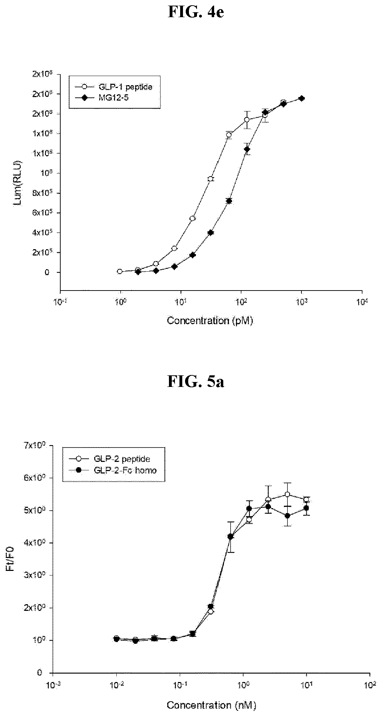

As a result of the analysis, as shown in Table 2 and FIGs. 4a to 4e, the GLP-1-Fc homodimer exhibited an activity of about 72% compared to the native GLP-1 peptide, and the bispecific fusion proteins according to Examples 1, 3, 4 and 5 of the present invention (MG12-1, 3, 4 and 5, respectively) showed relative activities of 9%, 118%, 39%, and 35%, respectively. An N-terminal mutation was introduced to prevent cleavage by the DPP-4 enzyme, and MG12-1 including a sugar chain in the hinge conjugated to GLP-1 was found to exhibit activity reduced by about 11 times due to glycosylation, and among MG12-1, MG12-3 having Exendin 4 introduced instead of GLP-1 showed activity increased by about 13 times compared to MG12-1. In MG12-1, MG12-4 and MG12-5 having introduced therein GLP-1/Exendin 4 hybrid that contains GLP-1 and Exendin 4, instead of GLP-1, showed similar relative activities at about 35-39% regardless of the presence of glycosylation of the hinge.

**Table 2**

| *In vitro* GLP-1 activity of bispecific fusion protein according to examples of the present invention | | | | | | |
|---|---|---|---|---|---|---|
| | GLP-1 peptide | GLP-1-Fc homodimer | Example 1 | Example 3 | Example 4 | Example 5 |
| EC₅₀ (pM) | 26 | 36 | 304 | 22 | 66 | 75 |
| vs. GLP-1 relative activity (%) | 100 | 72 | 9 | 118 | 39 | 35 |

### Experimental Example 2: Confirmation of GLP-2 in vitro activity

The present inventors investigated *in vitro* activity of GLP-2 of the bispecific fusion proteins prepared in Examples above by cAMP analysis.

Specifically, in order to evaluate the degree of cAMP induction by the GLP-2 specific reaction, a transformed cell line (Human GLP2R ACTOne^{™}), having GLP-2 receptor expressed in a cell line expressing CNG channels that open in a cAMP-specific manner, was procured. After the cells were thawed and properly maintained, the cells were separated from the flask under the same conditions as for the GLP-1 in vitro activity assay, and the number of cells required for activity evaluation was collected and washed. The washed cells were diluted with a cell culture medium (DMEM/high glucose medium, supplemented with 10% FBS, 5% G418, 0.01% puromycin) and seeded at 3~5×10⁴ cells/100 µL/well, and were incubated in an incubator for about 20 hours at 37°C, 5% CO₂. After removing the plate from the CO₂ incubator, the cells were observed under a microscope, and when the cell confluence reached 80% or more, 1X dye-loaded solution (Elite^{™} fluorescent membrane potential dye kit, eEnzyme) was added at 100 µL/well. While blocking the light at room temperature, the reaction was carried out for 2 to 2.5 hours, and before adding the test solutions, the fluorescence baseline (F₀) was measured using ELISA. Thereafter, the test solutions of various concentrations to be evaluated were treated at 50 µL/well, reacted for 0.5 hours, and then were measured for fluorescence value (Fₜ) using ELISA. The reactivity of each test solution was evaluated using the Fₜ/F₀ ratio.

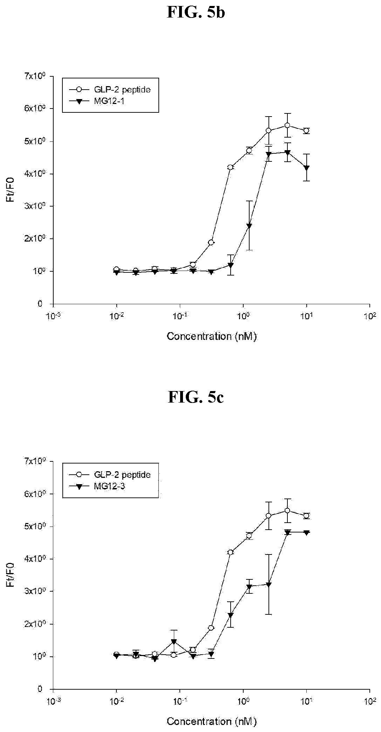

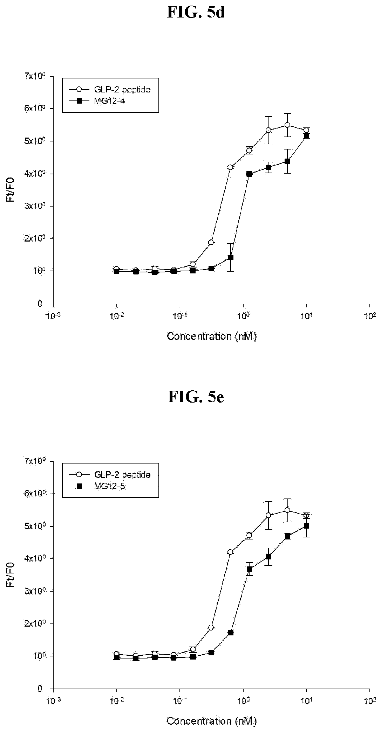

As a result, as shown in FIGs. 5a to 5e, the GLP-2-Fc homodimer exhibited an activity of about 132% compared to the native GLP-2 peptide, and the bispecific fusion proteins according to Examples 1, 3, 4 and 5 of the present invention (MG12-1, 3, 4 and 5, respectively) showed relative activities of 43%, 54%, 48%, and 59%, respectively. Since all MG12 variants have an N-terminal mutation introduced to prevent cleavage by the DPP-4 enzyme, and unlike GLP-1, since the hinge conjugated to GLP-2 does not contain a sugar chain, all of the variants showed similar GLP-2 activities.

**Table 3**

| *In vitro* GLP-2 activity of bispecific fusion proteins according to examples of the present invention | | | | | | |
|---|---|---|---|---|---|---|
| | GLP-2 peptide | GLP-2-Fc homodimer | Example 1 | Example 3 | Example 4 | Example 5 |
| EC₅₀(nM) | 0.58 | 0.44 | 1.35 | 1.07 | 1.22 | 0.99 |
| vs. GLP-2 relative activity (%) | 100 | 132 | 43 | 54 | 48 | 59 |

### Experimental Example 3: Analyses of in vitro GLP-1 and GLP-2 activity of MG12-2

The present inventors investigated how GLP-1 and GLP-2 activities differ in comparison to GLP-1-Fc homodimer and GLP-2-Fc homodimer, in case of the fusion proteins of Examples 1, 3, and 4, containing a glycan chain added to a hinge portion between GLP-1 and hybrid Fc region, and the bispecific fusion protein (MG12-2) of Example 2 designed such that a glycan chain is added to a hinge portion between GLP-2 and hybrid Fc region, while the fusion protein containing GLP-1 does not contain glycan chains.

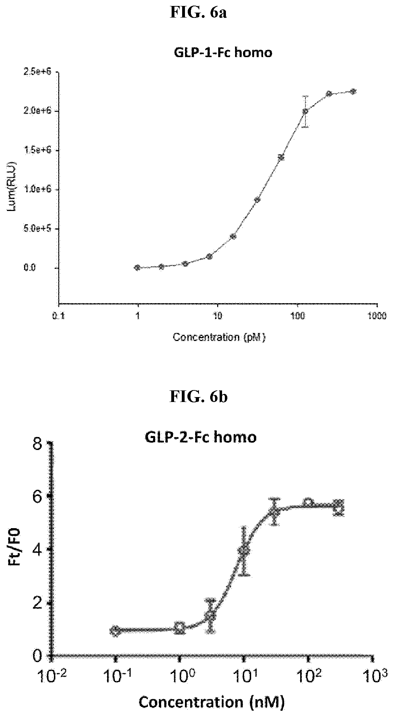

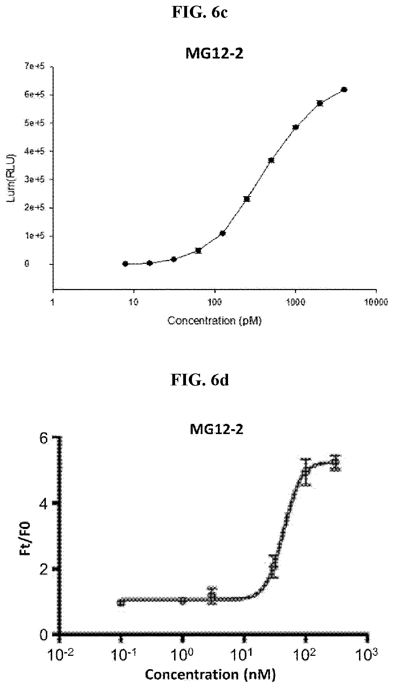

As a result, as shown in Table 4 and FIGs. 6a to 6d, for GLP-1 and GLP-2 activities, the bispecific fusion protein (MG12-2) of Example 2 of the present invention showed an activity of about 11.2% compared to GLP-1-Fc homodimer and an activity about 17.5% compared to GLP-2-Fc homodimer, respectively. This indicates that the GLP-1 activity was at a level similar to that of MG12-1, but the GLP-2 activity was about 4-5 times lower than that of other MG12-2 variants.

**Table 4**

| *In vitro* GLP-1 and GLP-2 activity of the bispecific fusion protein according to Example 2 of the present invention | | | | |
|---|---|---|---|---|
| | GLP-1 activity | | GLP-2 activity | |
| | GLP-1-Fc homodimer | MG12-2 | GLP-2-Fc homodimer | MG12-2 |
| EC₅₀ | 44.9 pM | 402.3 pM | 7.7 nM | 44 nM |
| relative activity (%) | 100 | 11.2 | 100 | 17.5 |

### Experimental Example 4: In vivo pharmacokinetics profile analysis

By comparing the half-life, area under the curve (AUC), highest blood concentration (Cₘₐₓ), etc. of the bispecific fusion proteins (MG12-1, 3, 4 and 5) according to Examples 1 and 3 to 5 prepared above, pharmacokinetic (PK) profiles were confirmed.

First, each protein was administered to 3 male SD (Sprague Dawley) rats per group at a dose of 1 mg/kg by subcutaneous (SC) route. Before injection and after 0.5, 1, 5, 10, 24, 48, 72, 120, and 168 hours after the injection, blood was collected and stored at room temperature for 30 minutes to aggregate. After centrifuging the aggregated blood at 3,000 rpm for 10 minutes, serum of each sample was collected and stored in a cryogenic freezer. Analyses were performed by an assay designed to specifically detect the GLP-1 site and Fc in the administered protein (GLP-1-Fc ELISA) and an assay designed to specifically detect the GLP-2 site and Fc in the administered protein (GLP-2-Fc ELISA). Specifically, a method of loading a biological sample on a plate coated with an antibody that binds to mouse-derived human immunoglobulin G4 (IgG4) to detect the target protein using a biotinylated anti-GLP-1 antibody (GLP-1-Fc ELISA), and a method of loading a biological sample on a plate coated with a monoclonal antibody that specifically reacts with GLP-2, to detect the target protein using a secondary antibody having HRP conjugated to mouse-derived human immunoglobulin G4 (IgG4) (GLP-2-Fc ELISA) were used. The obtained and prepared serum samples were loaded with appropriate dilutions to be analyzed at a position on a straight line of the standard curve.

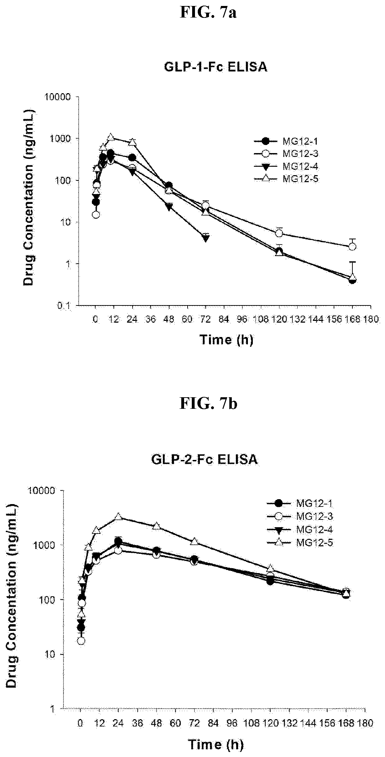

As a result, as shown in Tables 5 and 6 and FIGs. 7a and 7b, MG12-1, 3, 4, and 5 showed generally similar PK profiles to each other in the results analyzed by GLP-1-Fc ELISA and GLP-2-Fc ELISA. In terms of Cₘₐₓ, in both of the methods, MG12-5 showed the highest value whereas MG12-3 showed the lowest value. This trend was similarly observed in AUCₗₐₛₜ as well. In terms of the terminal half-life, MG12-3 showed the longest half-life in both of the methods, while MG12-4 showed the shortest half-life in the GLP-1-Fc ELISA method, and MG12-5 showed the shortest half-life in the GLP-2-Fc ELISA.

**Table 5**

| Examples | Cmax (ng/mL) | Tₘₐₓ (h) | AUCₗₐₛₜ (ng*h/mL) | Half-life (h) |
|---|---|---|---|---|
| 1 | 442.2±20.8 | 10 | 13919.8±824.9 | 16.5±6.3 |
| 3 | 301.0±57.4 | 10 | 9257.4±1543.3 | 29.5±2.7 |
| 4 | 328.7±34.5 | 10 | 7679.8±1228.9 | 9.0±0.3 |
| 5 | 1036.2±59.5 | 10 | 26059.6±3028.5 | 18.4±6.2 |

**Table 6**

| Examples | Cₘₐₓ (ng/mL) | Tₘₐₓ (h) | AUCₗₐₛₜ (ng*h/mL) | Half-life (h) |
|---|---|---|---|---|
| 1 | 1136.7±261.0 | 24.0±0.0 | 78639.0±11205.3 | 43.7±1.8 |
| 3 | 697.4±148.0 | 24.0±8.1 | 63977.3±10131.5 | 56.2±7.1 |
| 4 | 1052.5±112.8 | 24.0±0.0 | 78983.2±6542.8 | 49.0±2.1 |
| 5 | 3201.3±95.6 | 24.0±0.0 | 188741.6±7870.5 | 30.6±2.1 |

In conclusion, it was found that MG12-1, 3, 4, and 5 were adequately exposed when subcutaneously administered to normal model rats, and MG12-5 showed the highest Cₘₐₓ and AUCₗₐₛₜ.

### Experimental Example 5: Confirmation of in-vivo activity

### 5-1: Analyses on small intestine changes and nutrient absorption changes in experiment animal

In order to observe changes in the small intestine and nutrient absorption capacity following substance administration, an experiment was conducted in B6 normal mice. PBS and GLP-2-2G were administered twice a day, and GLP-2-Fc homodimer and MG12-5 were administered once every three days for 2 weeks. For the GLP-2-Fc homodimer and MG12-5 groups, PBS was administered twice a day, as was in the other administration groups, during the period when no drug was administered. Body weight and dietary intake were monitored at 3-day intervals, and feces were collected before administration, on days 5-6 after administration, and on days 11-12 after administration, and calorimetry was performed using a bomb calorimeter, and also, feed ingested during this period was also subjected to calorimetry through the same analysis. The energy absorption rate was defined as a value obtained by subtracting the calorific value of feces from the caloric value of ingested feed. On day 14 following drug administration, the experimental animals were sacrificed and the small intestine (pylorus ileum) was weighed.

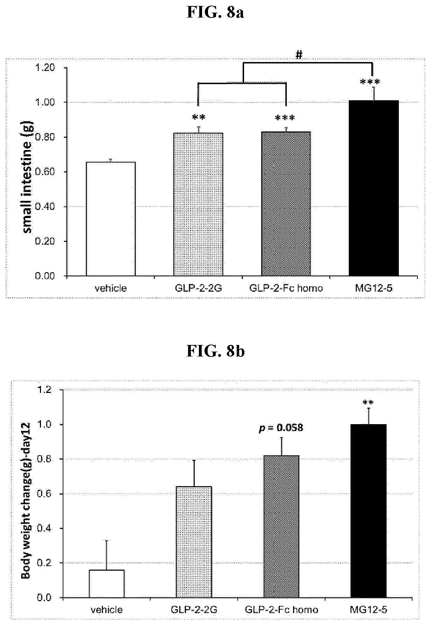

As a result, as shown in FIG. 8a, during the dosing period, an increase in small intestine weight was observed in other experimental groups compared to the control group, wherein the MG12-5 administered group showed the most notable increase in the small intestine weight, and as shown in FIGS. 8b, 8d, and 8e, this finding was consistent with a trend observed in body weight increases and energy absorption rate changes. In particular, as shown in FIG. 8d, in terms of change in the energy absorption rate after one week of administration, only the MG12-5 administered group according to an embodiment of the present invention showed a significant increasing trend. On the other hand, as shown in FIG. 8c, no change in energy absorption rate was observed in the experiments performed before administration of each test substance. However, there was a tendency of increased body weight in the drug-administered groups containing GLP-2, and it was found that the MG12-5 group showed an increase significant compared to the negative control group (vehicle-administered group). The energy absorption rate was found to be similar across all groups before administration, and in Week 1, which is after the second dose, there was an increasing trend in the groups administered with drugs containing GLP-2, but only MG12-5 showed a statistically significant increase compared to the vehicle-administered group (vehicle) and showed an increase even more significant than GLP-2-Fc homo in Week 2 after the fourth dose. After all drug administrations were complete, the small intestine weight of each group was measured. Similar to the energy absorption, a significant increase was observed in the groups administered with drugs containing GLP-2 compared to the control groups, and especially MG12-5 showed a statistically significant increase compared to the other two drug administered group.

In conclusion, MG12-5 was found to have a superior effect of increasing small intestine weight to GLP-2-2G and GLP-2-Fc homodimer, and this is considered to enable more efficient absorption of the calories of ingested feed, leading to a statistically significant body weight increase compared to other drug administered groups.

In addition, the present inventors conducted the same experiment as above while only varying the administered substance, in order to confirm whether the bispecific fusion proteins (MG12-1 and MG12-4) designed in a form other than MG12-5 also increase the small intestine weight and energy absorption rate of experimental animals. Specifically, PBS, 10 nmol/kg GLP-1-Fc homodimer, 10 nmol/kg GLP-2-Fc homodimer, 20 nmol/kg MG12-1, and 20 nmol/kg MG12-4 were subcutaneously administered. The drug was administered twice a week for a total of 4 times for 2 weeks. After 4 doses, the body weight was measured before autopsy, and the small intestine was excised after the autopsy and weighed. After the autopsy, the entire gastrointestinal tract of the mouse was separated, the mesentery and the pancreas were excised, and the area where the ileum and the cecum meet was cut to separate the small intestine and the large intestine. The separated small intestine was perfused and washed with 10% neutral formalin to remove the contents, and was weighed after removing the neutral formalin with an absorbent towel.

As a result of the experiment, as shown in FIGs. 9a and 9b, an increase in small intestine weight was observed in the GLP-1-Fc homodimer and GLP-2-Fc homodimer-administered groups, compared to the negative control group, and a statistically significant increase was observed in the GLP-2-Fc homodimer-administered group. The MG12-1 and MG12-4 administered groups showed a greater increase in small intestine weight than the GLP-2-Fc homodimer administered group, and both groups showed a statistically significant increase in small intestine weight compared to the negative control group. In terms of weight change before autopsy, there was a numerical decrease in GLP-1-Fc homodimer compared to the negative control group and there was no change in GLP-2-Fc homodimer, whereas a numerical increase in body weight was observed in both of the MG12-1 and MG12-4 administered groups. No statistically significant change was observed in all of the drug administered groups.

In conclusion, it was found that when administered to normal mice, the MG12-1 and MG12-4 administered groups showed a superior effect of increasing small intestine weight and increasing body weight compared to those of GLP-1-Fc homodimer and GLP-2-Fc homodimer.

### 5-2: Fluorescence Spectrometry

Next, the present inventors performed an experiment in B6 normal mice in order to observe the distribution in each tissue and organ according to the administration of a bispecific fusion protein according to an embodiment of the present invention. Cy5.5 was used as a fluorophore for tracking the distribution after administration of each substance. Buffer exchange was performed for each substance to be administered, MG12-1, MG12-4, and GLP-1Fc homodimer with boric acid buffer (pH 8.5), and with reference to the protocol of the Cy5.5 labeling kit (GE, #PA15605) were allowed to react with an appropriate amount of Cy5.5 overnight under refrigeration conditions. Unreacted Cy5.5 in the reaction-completed substance was removed through the PBS substitution process, and whether Cy5.5 was reacted well was confirmed through the final SDS-PAGE. Each administered substance was diluted with 10% DMSO/PBS to have the same fluorescence intensity using an ELISA plate reader capable of measuring fluorescence intensity before being administered. After drug administration, autopsy and perfusion were performed after 24 hours, estimated as Tmax, to compare and evaluate the distribution of each administered drug in the brain, small intestine, and large intestine.

As a result, as shown in Tables 7 and FIG. 10, the distribution of the three administered drugs in the brain did not show a significant difference, and the distribution in the small intestine and the large intestine were found to be larger in MG12-1 and MG12-4 compared to GLP-1-Fc homodimer, and this was a statistically significant difference.

In conclusion, when Cy5.5-conjugated GLP-1-Fc homodimer, MG12-1, MG12-4 were administered to normal mice, it was found that MG12-1 and MG12-4 were distributed in greater amounts in the small intestine and the large intestine compared to the GLP-1-Fc homodimer.

**Table 7**

| Average fluorescence intensity in each organ | | | |
|---|---|---|---|
| Substances | Brain | Small intestine | Large intestine |
| Cy5.5 | 0 | 0 | 0 |
| GLP-1-Fc homodimer | 161 | 854 | 310 |
| MG12-1 | 489 | 7320 | 3702 |
| MG12-4 | 297 | 4302 | 2343 |

### 5-3: PET-MRI analysis

Next, the present inventors performed an experiment in Balb/c-nude mice in order to observe the distribution in each tissue organ following the administration of a substance. Zr⁸⁹ was used as a radiotracer for tracking the distribution after administration of each substance. Each substance to be administered was labeled with Zr⁸⁹ as a reaction condition and a chelator selected through a preliminary test. The labeled Zr⁸⁹-protein was purified using a PD-10 column, and Zr⁸⁹ labeling efficiency and purity were confirmed using Radio TLC and SEC-HPLC. The labeling efficiency of the Zr⁸⁹-protein of 90% or more and the purity of 80% or more were determined to be appropriate, and the labeled protein was administered subcutaneously at 0.2 mCi/0.2 cc. Each administration substance was administered to three animals and static images for 20 minutes were obtained using PET-MRI at 1 hour, 6 hours, 24 hours, 48 hours, and 168 hours after injection. Through image analysis, the distribution of drugs as a function of time in the liver, small intestine, and large intestine was expressed as SUV (Standardized Uptake Value) and comparatively analyzed.

As a result of the analysis, as shown in FIG. 11, when only Fc was administered, there was no particularly distinguishable distribution characteristics between the three organs. For GLP-1-Fc homodimer and GLP-2-Fc homodimer, SUV values were increased compared to Fc in all of the three organs, and the increase was slightly larger in the small intestine and the large intestine in particular. In the case of MG12-1, MG12-4, and MG12-5, the distribution to the liver was reduced or showed no difference compared to Fc, and the distribution to the small intestine and large intestine were significantly high compared to that of GLP-1-Fc homodimer and GLP-2-Fc homodimer. This trend was more prevalent in MG12-1 and MG12-4.

In conclusion, the distribution of MG12-1, MG12-4, and MG12-5 was observed following the administration of Zr⁸⁹-attached Fc, GLP-1-Fc homodimer, GLP-2-Fc homodimer, MG12-1, MG12-4, and MG12-5 to Balb/c-nude mice. As a result, in MG12-1, MG12-4, and MG12-5, it was found that the distribution of substances to the small intestine and the large intestine was increased compared to that of Fc, GLP-1-Fc homo, and GLP-2-Fc homo.

### Experimental Example 6: Significant effect of MG12 vs. GLP-1 and GLP-2 combo

The present inventors endeavored to compare the effect of a bispecific fusion protein according to an embodiment of the present invention with a simple co-administration of GLP-1 and GLP-2.

In particular, in order to observe changes in body weight and the small intestine following the substance administration, an experiment was conducted in B6 normal mice. Subcutaneous administration of PBS (Vehicle), 10 nmol/kg Exendin-4 (Ex-4), 33.3 nmol/kg GLP-2-2G, Ex-4 + GLP-2-2G, 33.3 nmol/kg MG12-1, 33.3 nmol/kg MG12-3, 33.3 nmol/kg MG12-4, and 33.3 nmol/kg MG12-5 was conducted. For Ex-4, GLP-2-2G, and Ex-4 + GLP-2-2G, the drugs were administered twice a day for a total of 24 times for 12 days, and for the rest of the groups, the drugs were administered twice a week, for a total of 4 times for 12 days. After starting drug administration twice a week during the test period, feed intake was measured and expressed as cumulative feed intake, and pre-autopsy body weight was measured to calculate the body weight vs. feed intake. After the autopsy, the weight of the small intestine, and mucosal depth and villus height of the duodenum and the jejunum were measured. More specifically, after the autopsy, the entire gastrointestinal tract of the mouse was separated, the mesentery and the pancreas were excised, and the area where the ileum and the cecum meet was cut to separate the small intestine and the large intestine. The separated small intestine was perfused with 10% neutral formalin to remove the contents, and was weighed after removing the neutral formalin with an absorbent towel. From the weighed small intestine, the duodenum and jejunum portions were appropriately cut and fixed with 10% neutral formalin and used for histopathological examination.

As a result, compared to the control group (vehicle), Ex-4 and GLP-2-2G administered groups showed an increase in the small intestine weight, and all other experimental groups except Ex-4 showed a statistically significant increase in the small intestine weight compared to the control group (FIG. 12a). For the Ex-4 + GLP-2-2G administered group, the increase in the small intestine weight was numerically larger than when each substance was administered alone, and in the case of the MG12 according to an embodiment of the present invention, a numerically larger increase in the small intestine weight was observed compared to the co-administered group. In particular, the MG12-5 showed a statistically significant increase in the small intestine compared to the co-administered group. Similar tendencies were observed in the result of measurement of mucosal depth and villus height in the duodenum and the jejunum (FIGs. 12C and 12D). In order to confirm a body weight increase from the morphological increase of the small intestine, body weight increase tendencies were compared by normalization to the cumulative feed intake. As a result, as shown in FIG. 12b, there was a slight increase in Ex-4 and co-administered groups compared to the control group, and the MG12 showed a numerically greater body weight increase compared to the co-administered group, and in particular, MG12-3, MG12 -4 and MG12-5 showed a statistically significant body weight increase compared to the co-administered group.

In conclusion, when administered to normal mice, the group administered with the bispecific fusion protein (MG12) according to an embodiment of the present invention showed superior effects in terms of the small intestine weight, histopathological changes, and body weight changes normalized to feed intake compared to Ex-4 + GLP-2-2G.

### Experimental Example 7: Therapeutic effect of MG12 protein on metabolic syndrome

The present inventors endeavored to confirm whether MG12 according to an embodiment of the present invention has a therapeutic effect on various metabolic syndromes other than short bowel syndrome.

To this end, in particular, with respect to experimental animals induced with fatty liver and steatohepatitis through high-fat diet, as shown in FIG. 13a and Table 8, the present inventors performed a choline-deficient high-fat diet (CD-FHF) for 12 weeks using 8-week-old C57BL/6J male mice, and then 20 nmol/kg of MG12-5, MG12-8, and each of 10 nmol/kg of GLP-1 and GLP-2 combination (combo) were administrated twice subcutaneously twice to experimental animals respectively, along with a CD-HFD diet for 4 weeks, and various serological and histological analyses were performed.

**Table 8**

| Diet condition, administered substance, and dose for each experimental group | | | | | |
|---|---|---|---|---|---|
| Experimental group | Diet type | substances | Dose | Number of experiment animals | Admin. route |
| Normal diet group | Normal diet | PBS | - | 6 | SC |
| Negative control | CD-HFD | PBS | - | 6 | SC |
| MG12-8 administered group | CD-HFD | MG12-8 | 20 nmol/kg | 6 | SC |
| MG12-5 administered group | CD-HFD | MG12-5 | 20 nmol/kg | 6 | SC |
| GLP-1 and GLP-2 combination group | CD-HFD | GLP-1 + GLP-2 | 10 + 10 nmol/kg | 6 | SC |

### 7-1: Body weight change and food intake change analyses

First, the present inventors recorded changes in body weight and food intake of experimental animals during the experiment period. As a result, as shown in FIGs. 13b and 13c, the body weight of the experimental animals was significantly reduced when the MG12 of the present invention was administered, and in particular, the groups administered with MG12-5 and MG12-8 according to an embodiment of the present invention showed a greater relative body weight reduction effect compared to the normal diet group (NCD + PBS) and the GLP-1 and GLP-2 combo administered group, and as shown in FIG. 13D, showed a lower feed intake compared to the normal diet group or the combo-administered group. This suggests that the bispecific fusion protein according to an embodiment of the present invention has a superior effect than a simple co-administration of GLP-1 and GLP-2.

### 7-2: Blood glucose reducing effect analysis

Next, the present inventors investigated the blood glucose lowering effect in addition to the body weight reduction effect of MG-12 according to an embodiment of the present invention.

As described above, after 16 weeks on CD-HFD, mice treated with drugs (MG12-5, MG12-8, and GLP-1 and GLP-2 combo) twice a week for 4 weeks, after food restriction 12 hours, were intraperitoneally administered with glucose at a dose of 2 g/kg, and changes in blood glucose level were measured at a time interval for a total of 3 hours. As a result as shown in FIG. 14a, a higher increase in blood glucose level was observed in the CD-HFD diet group compared to the normal diet group. On the other hand, a statistically significant decrease in blood glucose level was observed in the MG12-5 and MG12-8 administered groups according to an embodiment of the present invention compared to the negative control group (CD-HFD + PBS) administered only with a vehicle. A statistically significantly higher efficacy was observed in MG12 compared to GLP-1 and GLP-2 co-administration. FIG. 14b is a graph showing the area under the curve related to changes in blood glucose level. The area under the curve of the change in blood glucose level increased by CD-HFD was decreased to a normal range by administration of GLP-1 and GLP-2, and in particular, was found to be decreased two times or more by MG12-5 and MG12-8.

### 7-3: Liver tissue examination

The present inventors sacrificed and extracted liver tissues from a mouse having completed the drug administration experiment as described above, and captured photographs thereof to examine changes in the size and color of the liver tissues, and measured the weight of the extracted liver tissues as well as ALT blood level, which is a measure of hepatotoxicity level.

As a result, as shown in FIG. 15a, a liver color similar to that of the normal diet group was observed in the MG12-5 administered group, the MG12-8 administered group, and the GLP-1 and GLP-2 co-administered group, and it was found that fat accumulation in liver tissues was decreased by the drugs. In addition, as shown in FIG. 15b, the liver weight between the normal diet group and the CD-HFD-fed group were similar, and the liver weight was reduced by MG12-5 and co-administration of GLP-1 and GLP-2, and the greatest decrease in liver weight was observed in the MG12-8 administered group. In addition, as shown in FIG. 15c, as a result of measuring a blood ALT level indicating liver toxicity after 16 weeks of CD-HFD diet, the ALT level increased approximately twice by CD-HFD diet, but the MG12-5 administered group and MG12-8 administered group showed a decrease in ALT level to a level similar to that of the normal diet group. On the other hand, the GLP-1 and GLP-2 co-administered group showed a high blood ALT level similar to that of the negative control group. This result indicates that while a simple co-administration of GLP-1 and GLP-2 had no hepatoprotective effect, MG12 according to an embodiment of the present invention, when administered, showed a hepatoprotective effect.

### Experimental Example 8: Therapeutic effect of MG12

### protein on non-alcoholic steatohepatitis

Based on the above results, the present inventors conducted an animal experiment using severe NASH-induced animal model to determine whether MG12 according to an embodiment of the present invention has a therapeutic effect on severe non-alcoholic steatohepatitis (NASH), which is a more advanced form of fatty liver.

To this end, in particular as illustrated in FIG. 16a, 2-day-old C57BL/6 mice were intraperitoneally administered with 200 mg/kg streptozotocin (STZ) once, and from the 4^{th} week was fed on a 60% high-fat diet for 4 weeks and then 60% high-fat diet for 3 weeks, and PBS (vehicle) as a negative control group, and MG12-8 drug according to an embodiment of the present invention was subcutaneously (s.c.) administered three times a week at doses of 10, 30, and 90 nmol/kg. At the 11^{th} week, the mice were sacrificed to obtain liver and blood, and analyses were performed. As a positive control group, OCA (obeticholic acid) was administered orally every day from the 8^{th} week at a concentration of 30 mg/kg for 3 weeks.

First, the result of the measurement of body weight changes as shown in FIG. 16b shows that both the positive control group and the experimental group treated with MG12-8 according to an embodiment of the present invention showed similar changes in body weight compared to the negative control group.

In addition, the present inventors measured blood AST and ALT concentrations, which are measures of liver damage, in the experimental animals. As a result, as shown in FIG. 16d, compared to the negative control group, the positive control group showed a similar level of expression for AST, and rather increased level for ALT compared to the negative control group. On the other hand, MG12-8 according to an embodiment of the present invention was found to statistically significantly lower both AST and ALT levels in blood regardless of the concentration. This indicates that MG12 according to an embodiment of the present invention is a substance having a therapeutic effect on severe NASH.

Next, the present inventors endeavored to analyze the therapeutic effect of MG12 according to an embodiment of the present invention on nonalcoholic fatty liver disease (NAFLD) through NAFLD activity index. The NAFLD activity index is an index expressed by comprehensively analyzing fat accumulation in liver, inflammatory response, and changes in the shape of hepatocytes. Fat accumulation is indicated in light green, inflammatory response is indicated in gray, and changes in hepatocyte (ballooning) is indicated in pink. As a result, as shown in FIG. 16e, compared to the negative control group, the positive control group slightly reduced fat accumulation in the liver, but failed to suppress inflammatory response, thus showing an overall index equal to that of the negative control group. On the other hand, it was confirmed that MG12-8 according to an embodiment of the present invention was found to lower the NAFLD activity index in a dose-dependent manner, and in particular, in the groups administered at 30 nmol/kg and 90 nmol/kg, showed a significant effect of lowering the index to 0.5 points or less, which is 1/8 or less than the negative control group. This result indicates that MG12 according to an embodiment of the present invention is a substance having an extremely effective drug candidate for NASH.

Based on the above results, it could be seen that MG12 according to an embodiment of the present invention is a very effective drug candidate for the treatment of metabolic diseases such as short bowel syndrome, obesity, type 2 diabetes, and non-alcoholic steatohepatitis, compared to a simple co-administration of GLP-1 and GLP-2. Accordingly, the heterodimeric fusion protein according to an embodiment of the present invention may be very usefully used as a therapeutic agent for various diseases that have been previously treated with GLP-1 and/or GLP-2, such as short bowel syndrome and metabolic diseases.

Although the present invention has been described with reference to the above-described examples and experimental examples, it will be understood that various modifications and equivalent other examples can be made possible therefrom by those skilled in the art. Therefore, the true technical protection scope of the present invention should be determined by the technical spirit of the appended claims.

### Industrial Applicability

A composition according to an embodiment of the present invention may be advantageously utilized as a therapeutic drug for metabolic diseases including obesity, type 2 diabetes, obesity, non-alcoholic fatty liver disease, non-alcoholic steatohepatitis, liver fibrosis, and the like.

## Claims

1. A bispecific fusion protein comprising a GLP-1 analogue and a GLP-2 analogue fused to each other.

2. The bispecific fusion protein of claim 1, further comprising a half-life extending moiety.

3. The bispecific fusion protein of claim 2, wherein the half-life extending moiety is inserted between the GLP-1 analogue and the GLP-2 analogue or added to an N-terminus or a C-terminus of the total fusion protein.

4. The bispecific fusion protein of claim 2, wherein the half-life extending moiety is an antibody Fc region, PEG, XTEN, PAS (Pro-Ala-Ser), ELP (elastin-like peptide), glycine-rich HAP (homo-amino-acid polymer), GLP (gelatin-like protein), or serum albumin.

5. A bispecific fusion protein comprising a first fusion protein having a GLP-1 analogue linked to an antibody Fc region, and a second fusion protein having a GLP-2 analogue linked to an antibody Fc region, wherein the bispecific fusion protein is generated by dimerization of the first fusion protein and the second fusion protein.

6. The bispecific fusion protein of any one of claims 1 to 5, wherein the GLP-1 analogue is GLP-1, Exendin 3, Exendin 4, GLP-1/Exendin 4 hybrid, GLP-1-XTEN, Lixisenatide, Albiglutide, Liraglutide, Dulaglutide, Extenatide, Taspoglutide, Lixisenatide, or a GLP-1 tandem repeat.

7. The bispecific fusion protein of claim 5, wherein at least one of the GLP-1 analogue and the GLP-2 analogue is a tandem repeat, and the GLP-1 analogue and the GLP-2 analogue have different numbers of repeats.

8. The bispecific fusion protein of claim 6, wherein the GLP-1 comprises an amino acid sequence represented by SEQ ID NO: 1 or 2.

9. The bispecific fusion protein of claim 6, wherein the Exendin 3 comprises an amino acid sequence represented by SEQ ID NO: 3.

10. The bispecific fusion protein of claim 6, wherein the Exendin 4 comprises an amino acid sequence represented by SEQ ID NO: 4.

11. The bispecific fusion protein of claim 6, wherein the GLP-1/Exendin 4 hybrid comprises an amino acid sequence represented by SEQ ID SEQ: 5.

12. The bispecific fusion protein of claim 6, wherein the Lixisenatide comprises an amino acid sequence represented by SEQ ID NO: 6.

13. The bispecific fusion protein of claim 6, wherein the Exendin 4-XTEN comprises an amino acid sequence represented by SEQ ID NO: 7.

14. The bispecific fusion protein of claim 6, wherein the Albiglutide comprises an amino acid sequence represented by SEQ ID NO: 8.

15. The bispecific fusion protein of claim 6, wherein the Liraglutide comprises an amino acid sequence represented by SEQ ID NO: 9.

16. The bispecific fusion protein of claim 6, wherein the Taspoglutide comprises an amino acid sequence represented by SEQ ID NO: 10.

17. The bispecific fusion protein of claim 6, wherein the GLP-1 tandem repeat comprises an amino acid sequence represented by SEQ ID NO: 11.

18. The bispecific fusion protein of claim 4 or 5, wherein the antibody Fc region is a hybrid antibody Fc region.

19. The bispecific fusion protein of claim 18, wherein the hybrid antibody Fc region is a Fc region in which at least two parts of two or more isotypes are mixed.

20. The bispecific fusion protein of claim 4 or 5,
wherein the first fusion protein comprises a modified hybrid Fc region in which 10^{th} amino acid, serine (S) is substituted with cysteine (C); and 22^{nd} amino acid, threonine (T) is substituted with tryptophan (W) in the CH3 domain (Knob structure), and the second fusion protein comprises a modified hybrid Fc region in which 5^{th} amino acid, tyrosine (Y) is substituted with cysteine (C); 22^{nd} amino acid, threonine (T) is substituted with serine (S); 24^{th} amino acid, leucine (L) is substituted with alanine (A); and 63^{rd} amino acid, tyrosine (Y) is substituted with valine (V) in the CH3 domain (Hole structure), or
wherein the first fusion protein comprises a modified hybrid Fc region in which 5^{th} amino acid, tyrosine (Y) is substituted with cysteine (C); 22^{nd} amino acid, threonine (T) is substituted with serine (S); 24^{th} amino acid, leucine (L) is substituted with alanine (A); and 63^{rd} amino acid, tyrosine (Y) is substituted with valine (V) in the CH3 domain (Hole structure), and the second fusion protein comprises a modified hybrid Fc region in which 10^{th} amino acid, serine (S) is substituted with cysteine (C); and 22^{nd} amino acid, threonine (T) is substituted with tryptophan (W) in the CH3 domain (Knob structure).

21. The bispecific fusion protein of claim 18, wherein the hybrid antibody Fc region comprises an amino acid sequence selected from the group consisting of SEQ ID NOs: 12 to 16.

22. The bispecific fusion protein of any one of claims 1 to 5, wherein the GLP-2 analogue is GLP-2, glepaglutide, or GLP-2 analogue 10.

23. The bispecific fusion protein of claim 22, wherein the GLP-2 comprises an amino acid sequence selected from the group consisting of SEQ ID NOs: 17 to 20.

24. The bispecific fusion protein of claim 22, wherein the Glepaglutide comprises an amino acid sequence represented by SEQ ID NO: 21.

25. The bispecific fusion protein of claim 22, wherein the GLP-2 analogue 10 comprises an amino acid sequence represented by SEQ ID NO: 22.

26. The bispecific fusion protein of claim 5, wherein the first fusion protein comprises an amino acid sequence selected from the group consisting of SEQ ID NOs: 23 to 30.

27. The bispecific fusion protein of claim 5, wherein the second fusion protein comprises an amino acid sequence selected from the group consisting of SEQ ID NOs: 31 to 36.

28. The bispecific fusion protein of claim 5, wherein a linker peptide is inserted between the GLP-1 analogue and the GLP-2 analogue, or between the GLP-1 analogue and the Fc region, or between the GLP-2 analogue and the Fc region.

29. The bispecific fusion protein of claim 28, wherein the linker peptide comprises or does not comprise an N-glycosylated site.

30. The bispecific fusion protein of claim 29, wherein the first fusion protein does not include an N-glycosylated site in the linker peptide, and the second fusion protein includes an N-glycosylated site in the linker peptide.

31. The bispecific fusion protein of claim 29, wherein the first fusion protein includes an N-glycosylated site in the linker peptide, and the second fusion protein does not include an N-glycosylated site in the linker peptide.

32. The bispecific fusion protein of claim 28, wherein the linker peptide is EPKSSDKTHTCPPCP (SEQ ID NO: 37), EPKSCDKTHTCPPCP (SEQ ID NO: 38), GGGGSGGGGSGGGGSEPKSSDKTHTCPPCP (SEQ ID NO: 39), GGGGSGGGGSGGGGSEPKSCDKTHTCPPCP (SEQ ID NO: 40), AKATTAPATTRNTGRGGEEKKKEKEKEEQEERETKTPECP (SEQ ID NO: 41), GGGGSGGGGSGGGGSEKEKEEQEERTHTCPPCP (SEQ ID NO: 42), GGGGSGGGGSGGGGSAKNTTAPATTRNTTRGGEEKKKEKEKEEQEERTHTCPPCP (SEQ ID NO: 43), AAGSGGGGGSGGGGSGGGGS (SEQ ID NO: 44), GGGGSGGGGSGGGGS (SEQ ID NO: 45), GGSGG (SEQ ID NO: 46), GGSGGSGGS (SEQ ID NO: 47), GGGSGG (SEQ ID NO: 48), SEQ ID NO: (G₄S)ₙ (subunit: SEQ ID NO: 49, n is an integer of 1 to 10), (GGS)ₙ (n is an integer of 1 to 10), (GS)ₙ (n is an integer of 1 to 10), (GSSGGS)ₙ (subunit: SEQ ID NO: 50, n is an integer of 1 to 10), KESGSVSSEQLAQFRSLD (SEQ ID NO: 51), EGKSSGSGSESKST (SEQ ID NO: 52), GSAGSAAGSGEF (SEQ ID NO: 53), (EAAAK)ₙ (subunit: SEQ ID NO: 54, n is an integer of 1 to 10), CRRRRRREAEAC (SEQ ID NO: 55), A(EAAAK)₄ALEA(EAAAK)₄A (SEQ ID NO: 56), GGGGGGGG (SEQ ID NO: 57), GGGGGG (SEQ ID NO: 58), AEAAAKEAAAAKA (SEQ ID NO: 59), PAPAP (SEQ ID NO: 60), (Ala-Pro)n (n is an integer of 1 to 10), VSQTSKLTRAETVFPDV (SEQ ID NO: 61, PLGLWA (SEQ ID NO: 62), TRHRQPRGWE (SEQ ID NO: 63), AGNRVRRSVG(SEQ ID NO: 64), RRRRRRRR(SEQ ID NO: 65), GFLG(SEQ ID NO: 66), GSSGGSGSSGGSGGGDEADGSRGSQKAGVDE (SEQ ID NO: 67), or GSTSGSGKPGSGEGS (SEQ ID NO: 68).

33. A pharmaceutical composition comprising the bispecific fusion protein of any one of claims 1 to 32 as an active ingredient.

34. The pharmaceutical composition of claim 33, which is used in treatment of a disease or symptom requiring intestinal proliferation.

35. The pharmaceutical composition of claim 34, wherein the disease or symptom requiring intestinal proliferation is malabsorption, an inflammatory bowel disease, or short bowel syndrome.

36. The pharmaceutical composition of claim 35, wherein the inflammatory bowel disease is ulcerative colitis, Behcet's disease, or short bowel syndrome.

37. A pharmaceutical composition for treating non-alcoholic steatohepatitis, comprising the bispecific fusion protein of any one of claims 1 to 32 as an active ingredient.

38. A pharmaceutical composition for treating metabolic syndrome, comprising the bispecific fusion protein of any one of claims 1 to 32 as an active ingredient.

39. A pharmaceutical composition for treating obesity, comprising the bispecific fusion protein of any one of claims 1 to 32 as an active ingredient.

40. A pharmaceutical composition for treating type 2 diabetes, comprising the bispecific fusion protein of any one of claims 1 to 32 as an active ingredient.

41. A pharmaceutical composition for treating liver fibrosis, comprising the bispecific fusion protein of any one of claims 1 to 32 as an active ingredient.

42. A method of treating a disease or symptom requiring intestinal proliferation in a subject with a disease requiring intestinal proliferation, the method comprising administering a therapeutically effective amount of the bispecific fusion protein of any one of claims 1 to 32 to the subject.

43. A method of treating a metabolic syndrome in a subject with metabolic syndrome, the method comprising administering a therapeutically effective amount of the bispecific fusion protein of any one of claims 1 to 32 to the subject.

44. A method of treating obesity in a subject with obesity, the method comprising administering a therapeutically effective amount of the bispecific fusion protein of any one of claims 1 to 32 to the subject.

45. A method of treating type 2 diabetes in a subject with type 2 diabetes, the method comprising administering a therapeutically effective amount of the bispecific fusion protein of any one of claims 1 to 32 to the subject.

46. A method of treating non-alcoholic fatty liver disease or non-alcoholic steatohepatitis in a subject with non-alcoholic fatty liver disease or non-alcoholic steatohepatitis, the method comprising administering a therapeutically effective amount of the bispecific fusion protein of any one of claims 1 to 32 to the subject.

47. A method of treating liver fibrosis in a subject with non-alcoholic liver fibrosis, the method comprising administering a therapeutically effective amount of the bispecific fusion protein of any one of claims 1 to 32 to the subject.
